(19) Europäisches Patentamt
European Patent Office
Office européen des brevets

(11) **EP 3 906 077 B1**

(12) **EUROPEAN PATENT SPECIFICATION**

(45) Date of publication and mention of the grant of the patent:
**04.03.2026 Bulletin 2026/10**

(21) Application number: **20736052.0**

(22) Date of filing: **03.01.2020**

(51) International Patent Classification (IPC):
*A61K 33/00* (2006.01)    *A61K 9/00* (2006.01)
*A61P 9/08* (2006.01)    *A61P 11/00* (2006.01)
*A61M 16/00* (2006.01)    *A61M 16/06* (2006.01)
*A61M 16/10* (2006.01)    *A61B 5/08* (2006.01)
*A61B 5/00* (2006.01)    *A61M 16/12* (2006.01)

(52) Cooperative Patent Classification (CPC):
(C-Sets available)
**A61B 5/0816; A61B 5/4839; A61B 5/7292;
A61K 9/0043; A61K 9/0073; A61K 33/00;
A61M 16/024; A61M 16/10; A61P 9/08;
A61P 11/00;** A61M 16/0672; A61M 16/12;
A61M 2016/0021; A61M 2016/0024;
A61M 2016/0027;    (Cont.)

(86) International application number:
**PCT/US2020/012138**

(87) International publication number:
**WO 2020/142658 (09.07.2020 Gazette 2020/28)**

(54) **USE OF INHALED NITRIC OXIDE (INO) FOR IMPROVING ACTIVITY LEVELS IN PATIENTS WITH LUNG-RELATED CONDITIONS**

VERWENDUNG VON INHALIERTEM STICKOXID (INO) ZUR VERBESSERUNG DES AKTIVITÄTSGRADES VON PATIENTEN MIT LUNGENERKRANKUNGEN

UTILISATION D'OXYDE NITRIQUE INHALÉ (INO) POUR AMÉLIORER LES NIVEAUX D'ACTIVITÉ CHEZ DES PATIENTS PRÉSENTANT DES AFFECTIONS PULMONAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.01.2019 US 201962788502 P
07.01.2019 US 201962789020 P
20.05.2019 US 201962850214 P**

(43) Date of publication of application:
**10.11.2021 Bulletin 2021/45**

(60) Divisional application:
**26154132.0**

(73) Proprietor: **Mallinckrodt Pharmaceuticals Ireland Limited
Dublin 15 (IE)**

(72) Inventors:
• **SHAH, Parag**
  **Morristown, NJ 07960 (US)**
• **FERNANDES, Peter Paul**
  **Randolph, NJ 07869 (US)**
• **KIM, Bobae**
  **Morristown, NJ 07960 (US)**
• **DEKKER, Martin**
  **East Califon, NJ 07830 (US)**

(74) Representative: **Boult Wade Tennant LLP
Salisbury Square House
8 Salisbury Square
London EC4Y 8AP (GB)**

(56) References cited:
**WO-A1-2019/222640    US-A1- 2018 304 038**

- BELLEROPHON PULSE TECHNOLOGIES: "NCT03267108 - A Safety and Efficacy Study of Pulsed Inhaled Nitric Oxide in Pulmonary Hypertension Associated With Pulmonary Fibrosis", INTERNET CITATION, 30 August 2017 (2017-08-30), pages 1 - 8, XP009521091, Retrieved from the Internet <URL:https://clinicaltrials.gov/ct2/show/NCT03267108>
- MELAO ALICE: "INOpulse Nitric-Oxide Dispenser Improves Lung Function in IPF Patients with Pulmonary Hypertension", PULMONARY HYPERTENSION NEWS, 3 May 2017 (2017-05-03), XP055722828, Retrieved from the Internet <URL:https://pulmonaryhypertensionnews.com/2017/05/03/inopulse-nitric-oxide-dispenser-improves-lung-functin-of-ph-ipf-patients>
- ANONYMOUS: "UNITED STATES SECURITIES AND EXCHANGE COMMISSION - FORM 8-K", BELLEROPHON, 28 April 2015 (2015-04-28), pages 1 - 56, XP055722832
- ISABEL BLANCO, RIBAS JESÚS, XAUBET ANTONI, GÓMEZ FEDERICO P., ROCA JOSEP, RODRIGUEZ-ROISIN ROBERT, BARBERÀ JOAN A.: "Effects of inhaled nitric oxide at rest and during exercise in idiopathic pulmonary fibrosis", JOURNAL OF APPLIED PHYSIOLOGY, vol. 110, no. 3, 23 December 2010 (2010-12-23), pages 639 - 645, XP055722836
- HAROLD R. COLLARD, ANSTROM KEVIN J., SCHWARZ MARVIN I., ZISMAN DAVID A.: "Sildenafil Improves Walk Distance in Idiopathic Pulmonary Fibrosis", CHEST, vol. 131, no. 3, March 2007 (2007-03-01) - 28 November 2007 (2007-11-28), pages 897 - 899, XP055722837

(52) Cooperative Patent Classification (CPC): (Cont.)
A61M 2016/1035; A61M 2202/0208;
A61M 2202/0275; A61M 2205/3303;
A61M 2205/332; A61M 2205/50; A61M 2230/06;
A61M 2230/205; A61M 2230/40; A61M 2230/63

C-Sets
A61M 2202/0208, A61M 2202/0007;
A61M 2202/0275, A61M 2202/0007;
A61M 2230/40, A61M 2230/005

**Description**

FIELD OF THE INVENTION

The present application relates to inhaled nitric oxide for use in methods for improving or maintaining activity levels in a patient in need thereof, in particular, pulsatile delivery of nitric oxide to patients with interstitial lung disease (ILD) to maintain and/or increase activity levels.

BACKGROUND OF THE INVENTION

Nitric oxide (NO) is a gas that, when inhaled, acts to dilate blood vessels in the lungs, improving oxygenation of the blood and reducing pulmonary hypertension. Because of this, nitric oxide is provided as a therapeutic gas in the inspiratory breathing phase for patients having shortness of breath (dyspnea) due to a disease state, for example, pulmonary arterial hypertension (PAH), chronic obstructive pulmonary disease (COPD), combined pulmonary fibrosis and emphysema (CPFE), cystic fibrosis (CF), idiopathic pulmonary fibrosis (IPF), emphysema, interstitial lung disease (ILD), chronic thromboembolic pulmonary hypertension (CTEPH), chronic high altitude sickness, or other lung disease.

While NO may be therapeutically effective when administered under the appropriate conditions, it can also become toxic if not administered correctly. NO reacts with oxygen to form nitrogen dioxide ($NO_2$), and $NO_2$ can be formed when oxygen or air is present in the NO delivery conduit. $NO_2$ is a toxic gas which may cause numerous side effects, and the Occupational Safety & Health Administration (OSHA) provides that the permissible exposure limit for general industry is only 5 ppm. Thus, it is desirable to limit exposure to $NO_2$ during NO therapy.

Blanco et al. (2011) J. Appl. Physiol. 110(3):638-645 (Published online on 23 December 2010) relates to the effects of inhaled nitric oxide at rest and during exercise in idiopathic pulmonary fibrosis.

The clinicaltrials.gov record of NCT03267108 available at https://clinicaltrials.gov/study/NCT03267108 and first posted on 30 August 2017 relates to a study to assess pulsed inhaled nitric oxide in subjects with pulmonary fibrosis at risk for pulmonary hypertension.

WO 2019/222640 relates to methods for pulsatile delivery of nitric oxide to a patient.

SUMMARY OF THE INVENTION

In one aspect, the present invention relates to inhaled nitric oxide (iNO) for use in a method for improving or maintaining activity levels in a patient in need thereof, wherein the method comprises administering iNO to the patient, wherein the patient has interstitial lung disease (ILD), and wherein the iNO is administered to the patient by: (a) detecting a breath pattern in the patient including a total inspiratory time; (b) correlating the breath pattern with an algorithm to calculate a timing of administration of a dose of nitric oxide; and (c) administering the dose of nitric oxide to the patient in a pulsatile manner over a portion of the total inspiratory time.

In some embodiments, the inhaled nitric oxide is administered at a dose in a range of from 25 micrograms (mcg)/kg ideal body weight (IBW)/hr to 50 mcg/kg IBW/hr. In some embodiments, the inhaled nitric oxide is administered at a dose in a range of from 30 mcg/kg IBW/hr to 45 mcg/kg IBW/hr. In certain embodiments, the inhaled nitric oxide is administered at a dose of 30 mcg/kg IBW/hr. In certain embodiments, the inhaled nitric oxide is administered at a dose of 45 mcg/kg IBW/hr.

In some embodiments, activity levels are improved or maintained as compared with a baseline activity level, wherein activity levels comprise one or more activity parameters selected from overall activity, non-sedentary activity, moderate activity, moderate to vigorous physical activity (MVPA), steps, calories, metabolic equivalent units (MET), sleep, heart rate, oxygen saturation, and calories burned. In some embodiments, the method further comprises measuring changes in activity levels using actigraphy.

In some embodiments, the algorithm uses one or both of a threshold sensitivity and a slope algorithm, wherein the slope algorithm detects a breath when the rate of pressure drop reaches a predetermined threshold.

In some embodiments, the interstitial lung disease (ILD) comprises one or more subtypes selected from idiopathic interstitial pneumonia (IIP), chronic hypersensitivity pneumonia, occupational or environmental lung disease, idiopathic pulmonary fibrosis (IPF), non-IPF IIPs, granulomatous, and connective tissue disease related ILD. In certain embodiments, the interstitial lung disease comprises idiopathic pulmonary fibrosis (IPF).

In some embodiments, the patient is at high risk of developing pulmonary hypertension.

A method of administering a dose of nitric oxide is described herein. In an embodiment of the invention, at least a single pulse dose is administered to a patient and is therapeutically effective to treat or alleviate symptoms of a pulmonary disease. In an embodiment of the invention, an aggregate of two or more pulse doses is therapeutically effective to treat or alleviate symptoms of a pulmonary disease.

In an embodiment of the invention, nitric oxide is delivered on a periodic basis for a minimum of five minutes per day to twenty-four hours per day. In an embodiment of the invention, the nitric oxide may be delivered at the convenience of the patient, for example, over a period of time while sleeping. In an embodiment of the invention, administration of pulses of nitric oxide may be evenly or unevenly spaced over a time period (e.g., ten minutes, one hour or for twenty-four hours). In another embodiment, administration of a therapeutically effective dose of nitric oxide may be continuous for a fixed period of time.

A method comprising detecting a breath pattern in a patient is described herein. In the present invention, the breath pattern includes the total inspiratory time (e.g., the time duration of a single inspiration of a patient). In an embodiment of the invention, the breath pattern is detected using a device comprising a breath sensitivity control. In the present invention, the breath pattern is correlated with an algorithm to calculate the timing of administration of a dose of nitric oxide. In an embodiment of the present invention, the volume of nitric oxide containing gas necessary for administration of an amount of nitric oxide on a per pulse basis is calculated. In the present invention, the nitric oxide is delivered to the patient in a pulsatile manner over a portion of a total inspiratory time.

In an embodiment of the invention, nitric oxide doses are delivered to the patient over a period of time sufficient to deliver a therapeutic dose of nitric oxide to the patient. In an embodiment of the invention, the device calculates the total time sufficient to deliver a therapeutic dose of nitric oxide to the patient. In an embodiment of the invention, the total time required for a therapeutic dose of nitric oxide to be delivered to the patient is at least partially dependent upon the breath pattern of said patient.

In an embodiment of the invention, nitric oxide is delivered during the first third of the total inspiratory time. In an embodiment, nitric oxide is delivered during the first half of the total inspiratory time. In an embodiment, nitric oxide is delivered during the first two-thirds of the total inspiratory time.

In an embodiment of the invention, at least fifty percent (50%) of the nitric oxide dose is delivered during the first third of the total inspiratory time. In an embodiment of the invention, at least seventy percent (70%) of the nitric oxide dose is delivered to the patient during the first half of the total inspiratory time. In an embodiment, at least ninety percent (90%) of the nitric oxide dose is delivered to the patient during the first two-thirds of the total inspiratory time. In an embodiment of the invention, at least ninety percent (90%) of the nitric oxide dose is delivered to the patient during the first third of the total inspiratory time. In an embodiment of the invention, all of the nitric oxide dose is delivered to the patient during the first half of the total inspiratory time.

In an embodiment of the invention, the breath sensitivity control on the device is adjustable. In an embodiment of the invention, the breath sensitivity control is fixed. In an embodiment of the invention, the breath sensitivity control is adjustable from a range of least sensitive to most sensitive, whereby the most sensitive setting is more sensitive at detecting breaths than the least sensitive setting.

Also described herein is a method for treating or alleviating symptoms of a cardiopulmonary disease. In an example of the disclosure, the method comprises detecting a breath pattern in a patient using a device comprising a breath sensitivity control. In an example of the disclosure, the breath pattern comprises a measurement of total inspiratory time. In an example of the disclosure, the breath pattern is correlated with an algorithm to calculate the timing of administration of a dose of nitric oxide. In an example of the disclosure, at least fifty percent (50%) of the dose of nitric oxide is delivered over the first third of the total inspiratory time. In an example of the disclosure, at least seventy percent (70%) of the dose of nitric oxide is delivered to the patient over the first half of the total inspiratory time. In an example of the disclosure, at least ninety percent (90%) of the dose of nitric oxide is delivered over the first two-thirds of the total inspiratory time.

In an embodiment of the invention, the device calculates the total time needed to deliver a therapeutically effective amount of nitric oxide to a patient. In an embodiment of the invention, the total time needed to deliver a therapeutically effective amount of nitric oxide is dependent upon one or more of a breath pattern, concentration of nitric oxide in a gas to be delivered to a patient, volume of a pulse dose, and duration of a pulse.

In an example of the disclosure, the pulmonary disease and/or lung related condition is selected from idiopathic pulmonary fibrosis (IPF), pulmonary fibrosis (PF), interstitial lung disease (ILD), pulmonary arterial hypertension (PAH), chronic obstructive pulmonary disorder (COPD), cystic fibrosis (CF), emphysema, combined pulmonary fibrosis and emphysema (CPFE), chronic thromboembolic pulmonary hypertension (CTEPH), chronic high altitude sickness, or other lung disease. In an example of the disclosure, the pulmonary disease is pulmonary hypertension associated with other pulmonary diseases such as Group I-V pulmonary hypertension (PH). In an embodiment of the invention, the pulmonary disease and/or lung-related condition is pulmonary hypertension associated with interstitial lung disease. In an example of the disclosure, the pulmonary disease and/or lung-related condition is pulmonary hypertension associated with pulmonary fibrosis. In an embodiment of the invention, the pulmonary disease and/or lung-related condition is pulmonary hypertension associated with idiopathic pulmonary fibrosis. In an embodiment of the invention, a patient suffering from ILD is at high risk of developing pulmonary hypertension. In another embodiment of the invention, a patient suffering from ILD is at low risk of developing pulmonary hypertension. In an embodiment of the invention, a patient suffering from ILD is at medium risk of developing pulmonary hypertension. In an embodiment of the invention, a patient suffering from IPF is at high risk of developing pulmonary hypertension. In an embodiment of the invention, a patient suffering from IPF is at medium risk of developing pulmonary hypertension. In another embodiment of the invention, a patient suffering from IPF is at low risk of developing pulmonary hypertension. In an example of the disclosure, a patient suffering from PF is at high risk of developing pulmonary hypertension. In an example of the disclosure, a patient suffering from PF is at medium risk of developing pulmonary hypertension. In an example of the disclosure, a patient suffering from PF is at low risk of developing pulmonary hypertension.

A programmable device for delivering a dose of nitric oxide is also described herein. In an example of the disclosure, the device comprises a nasal delivery portion, a drug cartridge comprising nitric oxide, an oxygen source, a breath sensitivity portion to detect breath patterns in the patient, a breath detection algorithm for determining the dose of nitric oxide delivered to the patient, and a portion for administering the dose of nitric oxide to the patient through a series of pulses that correlate with the inspiratory portion of the breath pattern. In an example of the disclosure, the breath sensitivity portion of the device comprises an adjustable or fixed breath sensitivity setting. In an example of the disclosure, the nasal delivery portion is a nasal cannula, a face mask, an atomizer, or a nasal inhaler. In an embodiment of the invention, the breath detection algorithm uses a threshold sensitivity and a slope algorithm. In an embodiment of the invention, the slope algorithm counts a breath as detected when the rate of pressure drop reaches a threshold level.

In an embodiment, iNO for use in a method of the invention maintains or increases activity levels in patients with pulmonary hypertension, wherein the patient's pulmonary hypertension is associated with interstitial lung disease. In an embodiment of the invention, the iNO is administered continuously in a pulsatile manner for a period of at least 12 hours, at least 13 hours, at least 14 hours, at least 15 hours, at least 16 hours, at least 17 hours, at least 18 hours, at least 19 hours, at least 20 hours, at least 21 hours, at least 22 hours, at least 23 hours, or at least 24 hours. In another embodiment, the iNO is administered at 30 mcg/kg IBW/hr. In another embodiment, the iNO is administered at 45 mcg/kg IBW/hr. In another embodiment, the iNO is administered at 75 mcg/kg IBW/hr. In an embodiment of the invention, the iNO is administered in combination with supplemental oxygen.

The methods described herein may comprise administering iNO by first detecting a breath pattern in said patient including a total inspiratory time; correlating the breath pattern with an algorithm to calculate the timing of administration of the dose of nitric oxide; and administering the dose of nitric oxide to said patient in a pulsatile manner over a portion of the total inspiratory time.

Also described herein is a method for treating pulmonary hypertension associated with interstitial lung disease. In an example of the disclosure, the method comprises administering inhaled nitric oxide to the patient.

In an embodiment, iNO for use in a method of the invention prevents a decline in activity levels in patients with pulmonary hypertension associated with interstitial lung disease.

In an embodiment of the invention, actigraphy is used to measure activity parameters.

In an embodiment of the invention, iNO is for use in a method for improving activity levels in a patient with pulmonary hypertension associated with a lung condition selected from the group consisting of interstitial lung disease and idiopathic pulmonary fibrosis, the method comprising administering inhaled nitric oxide to said patient, wherein inhaled nitric oxide is administered by detecting a breath pattern in said patient including a total inspiratory time; correlating the breath pattern with an algorithm to calculate the timing of administration of the dose of nitric oxide; and administering the dose of nitric oxide to said patient in a pulsatile manner over a portion of the total inspiratory time.

Also described herein is a method for treating pulmonary hypertension associated with a lung condition selected from the group consisting of interstitial lung disease, idiopathic pulmonary fibrosis, and pulmonary fibrosis, the method comprising administering inhaled nitric oxide to said patient. In an example of the disclosure, inhaled nitric oxide is administered by detecting a breath pattern in said patient including a total inspiratory time; correlating the breath pattern with an algorithm to calculate the timing of administration of the dose of nitric oxide; and administering the dose of nitric oxide to said patient in a pulsatile manner over a portion of the total inspiratory time.

In an embodiment of the invention, iNO is for use in a method for preventing a decline in activity levels in a patient with pulmonary hypertension associated with a lung condition selected from the group consisting of interstitial lung disease and idiopathic pulmonary fibrosis, the method comprising administering inhaled nitric oxide to said patient, wherein inhaled nitric oxide is administered by detecting a breath pattern in said patient including a total inspiratory time; correlating the breath pattern with an algorithm to calculate the timing of administration of the dose of nitric oxide; and administering the dose of nitric oxide to said patient in a pulsatile manner over a portion of the total inspiratory time.

In an embodiment of the invention, iNO is for use in a method for maintaining activity levels in a patient with pulmonary hypertension associated with a lung condition selected from the group consisting of interstitial lung disease and idiopathic pulmonary fibrosis, the method comprising administering inhaled nitric oxide to said patient, wherein inhaled nitric oxide is administered by detecting a breath pattern in said patient including a total inspiratory time; correlating the breath pattern with an algorithm to calculate the timing of administration of the dose of nitric oxide; and administering the dose of nitric oxide to said patient in a pulsatile manner over a portion of the total inspiratory time.

In an embodiment of the invention, iNO is for use in a method for improving activity levels in a patient with a lung condition selected from the group consisting of interstitial lung disease and idiopathic pulmonary fibrosis, the method comprising administering inhaled nitric oxide to said patient, wherein inhaled nitric oxide is administered by detecting a breath pattern in said patient including a total inspiratory time; correlating the breath pattern with an algorithm to calculate the timing of administration of the dose of nitric oxide; and administering the dose of nitric oxide to said patient in a pulsatile manner over a portion of the total inspiratory time.

Also described herein is a method for preventing a decline in activity levels in a patient with a lung condition, the method comprising administering inhaled nitric oxide to said patient. Also described herein is a method for maintaining activity levels in a patient with a lung condition, the method comprising administering inhaled nitric oxide to said patient. Also described herein is a method for improving activity levels in a patient with pulmonary hypertension associated with a lung condition selected from the group consisting of interstitial lung disease, idiopathic pulmonary fibrosis, and pulmonary fibrosis, the method comprising administering a vasodilator to said patient. In one example of the disclosure, the vasodilator is a systemic vasodilator. In another example of the disclosure, the vasodilator is a local acting vasodilator. In the present invention, the vasodilator is inhaled nitric oxide.

In an embodiment of the invention, the inhaled nitric oxide is administered in a range of from about 25 mcg/kg IBW/hr to about 50 mcg/kg IBW/hr. In another embodiment, the inhaled nitric oxide is administered at a dose in a range of from about 30 mcg/kg IBW/hr to about 45 mcg/kg IBW/hr. In another embodiment, the inhaled nitric oxide is administered at a dose of about 30 mcg/kg IBW/hr. In another embodiment, the inhaled nitric oxide is administered at a dose of about 45 mcg/kg IBW/hr.

Various embodiments are listed above and will be described in more detail below. It will be understood that the embodiments listed may be combined not only as listed below, but in other suitable combinations in accordance with the scope of the invention.

The foregoing has outlined rather broadly certain features and technical advantages of the present invention. It should be appreciated by those skilled in the art that the specific embodiments disclosed may be readily utilized as a basis for modifying or designing other structures or processes within the scope present invention.

BRIEF DESCRIPTION OF THE DRAWINGS

The foregoing summary, as well as the following detailed description of the invention, will be better understood when read in conjunction with the appended drawings.

So that the manner in which the above recited features of the present invention can be understood in detail, a more particular description of the invention, briefly summarized above, may be had by reference to embodiments, some of which are illustrated in the appended drawings. It is to be noted, however, that the appended drawings illustrate only typical embodiments of this invention and are therefore not to be considered limiting of its scope, for the invention may admit to other equally effective embodiments.

FIG. 1 is a graph demonstrating a single measurement of a breath.

FIG. 2 is a graph demonstrating measurement of a delivered pulse of nitric oxide to a patient according to the present invention.

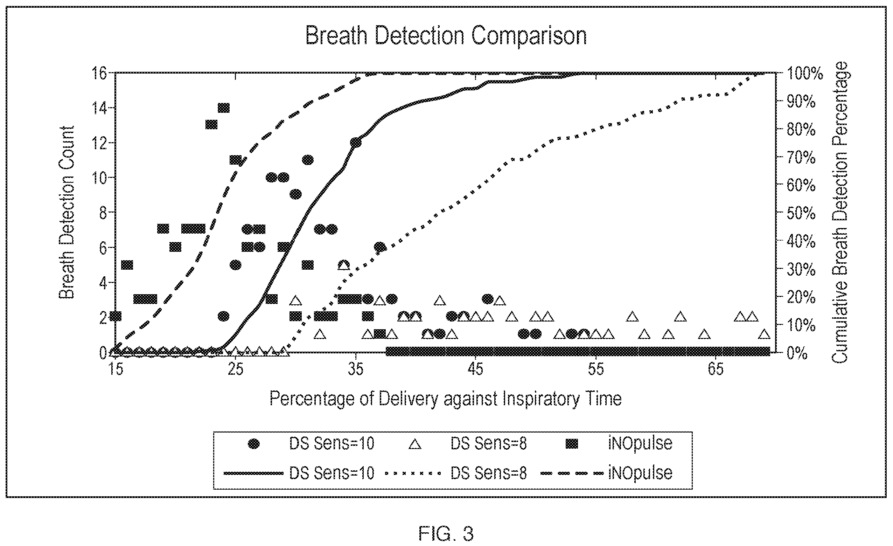

FIG. 3 is a graph demonstrating detection of breaths as a percentage of nitric oxide delivery over total inspiratory time. The orange line represents a breath sensitivity setting of 8 of 10 (e.g., 80% of maximum sensitivity) on Device 1, the blue line represents a breath sensitivity setting of 10 of 10 (e.g., maximum sensitivity) on Device 1, and the green line represents a fixed breath sensitivity setting of 10 on Device 2. The green line demonstrates that about 93% of the nitric oxide dose is delivered during the first 33% (or first third) of total inspiratory time, and 100% of the nitric oxide dose is delivered during the first 50% (or first half) of total inspiratory time. The blue line demonstrates that about 62% of the nitric oxide dose is delivered during the first 33% (or first third) of total inspiratory time, about 98% is delivered during the first 50% (or first half) of total inspiratory time, and 100% is delivered during the first 67% (or first two-thirds) of total inspiratory time. The orange line demonstrates that about 17 % of the nitric oxide dose is delivered during the first 33% (or first third) of total inspiratory time, about 72% is delivered during the first 50% (or first half) of total inspiratory time, and about 95% during the first 67% (or first two-thirds) of total inspiratory time.

FIG. 4 depicts the combined results described in FIG. 3.

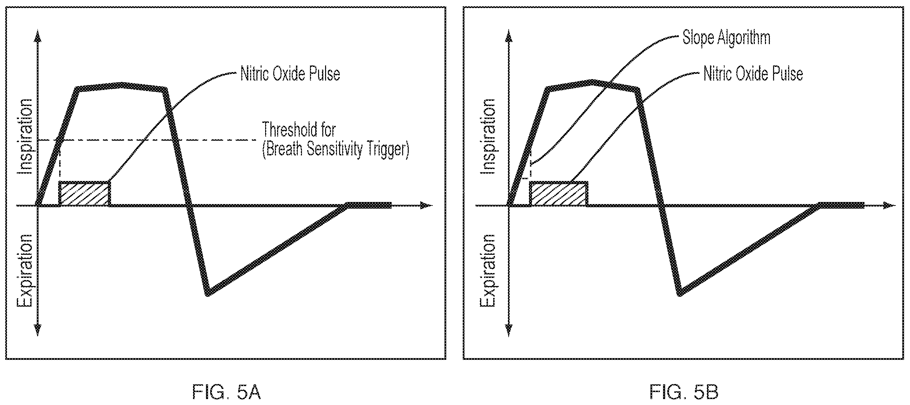

FIGS. 5A and 5B depict an algorithm for breath detection and delivery of nitric oxide. FIG. 5A demonstrates a threshold algorithm. FIG. 5B demonstrates a slope algorithm.

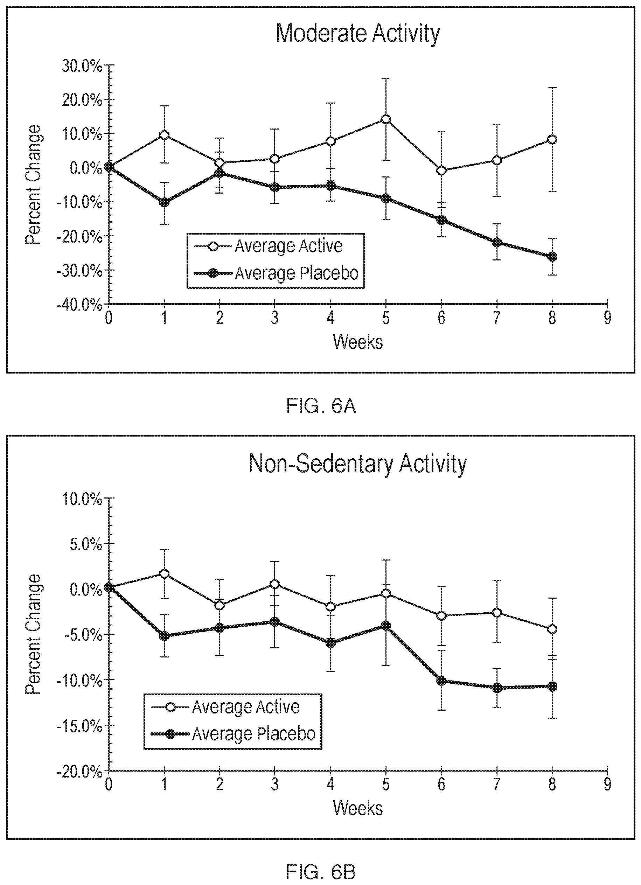

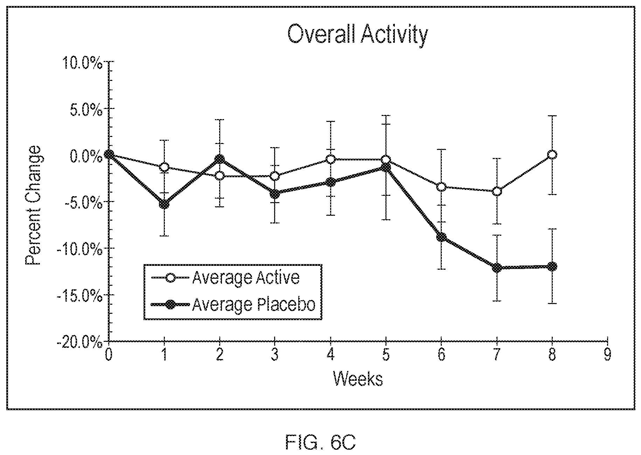

FIGS. 6A-6C show percent change over time for activity parameters in iNO treated patients and placebo patients. FIG. 6A shows percent change in moderate activity, FIG. 6B shows percent change in non-sedentary activity, and FIG. 6C shows percent change in overall activity.

FIGS. 7A-7D show percent change over time for activity parameters in iNO treated patients and placebo patients. FIG. 7A shows percent change in moderate to vigorous physical activity (MVPA) activity, FIG. 7B shows percent change in overall activity, FIG. 7C shows percent change in non-sedentary activity, and FIG. 7D shows percent change in daily calories intake.

FIGS. 8A and 8B illustrate comparative data for average weekly change in MVPA (FIG. 8A) and overall activity (FIG. 8B) from the blinded portion of the study as compared with the open label extension (OLE) portion of the study.

FIGS. 9A and 9B illustrate Cohort 2 (iNO45) comparative data for normalized MVPA change from baseline over months 1-4 (FIG. 9A) and overall activity change from baseline over months 1-4 (FIG. 9B). In FIG. 9A, baseline MVPA was 74 min/day, and an improvement of 14 min/day was seen at month 4. In FIG. 9B, baseline overall activity was 1476 counts per minute, and an improvement of 100 counts per minute was seen at month 4.

FIGS. 10A-10C illustrate Cohort 2 (iNO45) comparative data on the St. George's Respiratory Questionnaire (SGRQ) at month 4. The SGRQ is an inverse scale, so higher point totals indicate a worsening status. FIG. 10A illustrates a 3 point improvement in the SGRQ total, which measures health status and quality of life. FIG. 10B illustrates a 5 point improvement in SGRQ Activity, which measures disturbances to a patient's physical activity. FIG. 10C illustrates a 6 point improvement in the SGRQ Impacts, which measures psychological and social impact of disease/condition.

FIG. 11 illustrates Cohort 2 (iNO45) comparative data on the University of California, San Diego (UCSD) Shortness of Breath Questionnaire (SOBQ), which indicates benefit in dyspnea. Here again, an increased score indicates worsening of the disease. A 5 point improvement is shown, which measures shortness of breath while patients perform daily physical activity.

FIG. 12 illustrates the log transformed estimated change in MVPA (FIG. 12A) and Overall Activity (FIG. 12B) at Month 2 (for iNO30 and iNO45, Cohorts 1 and 2) and Month 4 (for iNO45, Cohort 2).

FIG. 13 illustrates the log transformed MVPA predicted marginal effects on a monthly (FIG. 13A) and weekly (FIG. 13B) basis for Cohort 2.

## DETAILED DESCRIPTION OF THE INVENTION

Unless defined otherwise, all technical and scientific terms used herein have the same meaning as is commonly understood by one of skill in the art to which this invention belongs.

Before describing several exemplary embodiments of the invention, it is to be understood that the invention is not limited to the details of construction or process steps set forth in the following description. The invention is capable of other embodiments and of being practiced or being carried out in various ways.

Reference throughout this specification to "one embodiment," "certain embodiments," "one or more embodiments" or "an embodiment" means that a particular feature, structure, material, or characteristic described in connection with the embodiment is included in at least one embodiment of the invention. Thus, the appearances of the phrases such as "in one or more embodiments," "in certain embodiments," "in one embodiment" or "in an embodiment" in various places throughout this specification are not necessarily referring to the same embodiment of the invention. Furthermore, the particular features, structures, materials, or characteristics may be combined in any suitable manner in one or more embodiments.

Although the invention herein has been described with reference to particular embodiments, it is to be understood that these embodiments are merely illustrative of the principles and applications of the present invention. Thus, it is intended that the present invention include modifications and variations that are within the scope of the appended claims and their equivalents.

Definitions

The term "effective amount" or "therapeutically effective amount" refers to that amount of a compound or combination of compounds as described herein that is sufficient to effect the intended application including, but not limited to, disease treatment. A therapeutically effective amount may vary depending upon the intended application (*in vitro* or *in vivo*), or the subject and disease condition being treated (*e.g.,* the weight, age and gender of the subject), the severity of the disease condition, the manner of administration, *etc.* which can readily be determined by one of ordinary skill in the art. The term also applies to a dose that will induce a particular response in target cells (*e.g.,* the reduction of platelet adhesion and/or cell migration). The specific dose will vary depending on the particular compounds chosen, the dosing regimen to be followed, whether the compound is administered in combination with other compounds, timing of administration, the tissue to which it is administered, and the physical delivery system in which the compound is carried.

A "therapeutic effect" as that term is used herein, encompasses a therapeutic benefit and/or a prophylactic benefit. A prophylactic effect includes delaying or eliminating the appearance of a disease or condition, delaying or eliminating the onset of symptoms of a disease or condition, slowing, halting, or reversing the progression of a disease or condition, or any combination thereof.

The disease state of "interstitial lung disease" or "ILD" shall include all subtypes of ILD, including, but not limited to, idiopathic interstitial pneumonia (IIP), chronic hypersensitivity pneumonia, occupational or environmental lung disease, idiopathic pulmonary fibrosis (IPF), non-IPF IIPs, granulomatous (e.g., sarcoidosis), connective tissue disease related ILD, and other forms of ILD.

When ranges are used herein to describe an aspect of the present invention, for example, dosing ranges, amounts of a component of a formulation, etc., all combinations and subcombinations of ranges and specific embodiments therein are intended to be included. Use of the term "about" when referring to a number or a numerical range means that the number or numerical range referred to is an approximation within experimental variability (or within statistical experimental error), and thus the number or numerical range may vary. The variation is typically from 0% to 15%, preferably from 0% to 10%, more preferably from 0% to 5% of the stated number or numerical range. The term "comprising" (and related terms such as "comprise" or "comprises" or "having" or "including") includes those embodiments such as, for example, an embodiment of any composition of matter, method or process that "consist of" or "consist essentially of" the described features.

For the avoidance of doubt, it is intended herein that particular features (for example integers, characteristics, values, uses, diseases, formulae, compounds or groups) described in conjunction with a particular aspect, embodiment or example of the invention are to be understood as applicable to any other aspect, embodiment or example described herein unless incompatible therewith. Thus such features may be used where appropriate in conjunction with any of the definition, claims or embodiments defined herein. All of the features disclosed in this specification (including any accompanying claims, abstract and drawings), and/or all of the steps of any method or process so disclosed, may be combined in any combination, except combinations where at least some of the features and/or steps are mutually exclusive. The invention is not restricted to any details of any disclosed embodiments. The invention extends to any novel one, or novel combination, of the features disclosed in this specification (including any accompanying claims, abstract and drawings), or to any novel one, or any novel combination, of the steps of any method or process so disclosed.

Effective dosing of NO is based on a number of different variables, including quantity of drug and the timing of delivery. Several patents have been granted relating to NO delivery, including US Patent Nos. 7,523,752; 8,757,148; 8,770,199; and 8,803,717, and a Design Patent D701,963 for a design of an NO delivery device. Additionally, there are pending applications relating to delivery of NO, including US2013/0239963 and US2016/0106949. Even in view of these patents and pending publications, there is still a need for methods and apparatuses that deliver NO in a precise, controlled manner, so as to maximize the benefit of a therapeutic dose and minimize the potentially harmful side effects.

With respect to the present invention, a dose of NO is administered in a pulse to a patient during an inspiration by the patient in accordance with the claims. It has been surprisingly discovered that nitric oxide delivery can be precisely and accurately delivered within the first two-thirds of total breath inspiration time and the patient obtains benefits from such delivery. Such delivery minimizes loss of drug product and risk of detrimental side effects increases the efficacy of a pulse dose which in turn results in a lower overall amount of NO that needs to be administered to the patient in order to be effective. Such delivery is useful for the treatment of various diseases, such as but not limited to idiopathic pulmonary fibrosis (IPF), pulmonary arterial hypertension (PAH), including Groups I-V pulmonary hypertension (PH), chronic obstructive pulmonary disorder (COPD), cystic fibrosis (CF), and emphysema, and is also useful as an antimicrobial, for example, in treating pneumonia.

Such precision has further advantages in that only portions of the poorly ventilated lung area is exposed to NO. Hypoxia and issues with hemoglobin may also be reduced with such pulsed delivery, while $NO_2$ exposure is also more limited.

A Device of the Present Disclosure

In certain examples, the present disclosure includes a device, e.g. a programmable device for delivering a dose of a gas (e.g., nitric oxide) to a patient in need. The device can include a delivery portion, a drug cartridge including a compressed gas for delivery to a patient, a breath sensitivity portion to detect a breath pattern in patient comprising a breath sensitivity setting, at least one breath detection algorithm for determining when to administer the compressed gas to the patient and a portion for administering the dose of nitric oxide to the patient through a series of one or more pulses.

In certain examples of the disclosure, the drug cartridge is replaceable.

In certain examples of the disclosure, the delivery portion includes one or more of a nasal cannula, a face mask, an atomizer, and a nasal inhaler. In certain examples of the disclosure, the delivery portion can further include a second delivery portion to permit the simultaneous administration of one or more other gases (e.g, oxygen) to a patient.

In certain examples of the disclosure, and as detailed elsewhere herein, the device includes an algorithm wherein the algorithm uses one or both of a threshold sensitivity and a slope algorithm, wherein the slope algorithm detects a breath when the rate of pressure drop reaches a predetermined threshold.

In an example of the disclosure, mechanically, a pulse dose of a gas can reduce, if not eliminate, venturi effects which would normally create problems for other gas sensors. For example, in the absence of the pulse doses of the present disclosure, $O_2$ back pressure sensors may override delivery of $O_2$ when $O_2$ is administered simultaneously with another gas such as NO.

Breath Patterns, Detection and Triggers

Breath patterns vary based on the individual, time of day, level of activity, and other variables; thus it is difficult to predetermine a breath pattern of an individual. A delivery system that delivers therapeutics to a patient based on breath pattern, then, should be able to handle a range of potential breath patterns in order to be effective.

In certain embodiments, the patient or individual can be any age, however, in more certain embodiments the patient is sixteen years of age or older.

In the invention, the breath pattern includes a measurement of total inspiratory time, which as used herein is determined for a single breath. However, depending on context "total inspiratory time" can also refer to a summation of all inspiratory times for all detected breaths during a therapy. Total inspiratory time may be observed or calculated. In another embodiment, total inspiratory time is a validated time based on simulated breath patterns.

In an embodiment of the invention, breath detection includes at least one and in some embodiments at least two separate triggers functioning together, namely a breath level trigger and/or a breath slope trigger.

In an embodiment of the invention, a breath level trigger algorithm is used for breath detection. The breath level trigger detects a breath when a threshold level of pressure (e.g., a threshold negative pressure) is reached upon inspiration.

In an embodiment of the invention, a breath slope trigger detects breath when the slope of a pressure waveform indicates inspiration. The breath slope trigger is, in certain instances, more accurate than a threshold trigger, particularly when used for detecting short, shallow breaths.

In an embodiment of the invention, a combination of these two triggers provides overall a more accurate breath detection system, particularly when multiple therapeutic gases are being administered to a patient simultaneously.

In an embodiment of the invention, the breath sensitivity control for detection of either breath level and/or breath slope is fixed. In an embodiment of the invention, the breath sensitivity control for detection of either breath level or breath slope is adjustable or programmable. In an embodiment of the invention, the breath sensitivity control for either breath level and/or breath slope is adjustable from a range of least sensitive to most sensitive, whereby the most sensitive setting is more sensitive at detecting breaths than the least sensitive setting.

In certain embodiments where at least two triggers are used, the sensitivity of each trigger is set at different relative levels. In one embodiment where at least two triggers are used, one trigger is set a maximum sensitivity and another trigger is set at less than maximum sensitivity. In one embodiment where at least two triggers are used and where one trigger is a breath level trigger, the breath level trigger is set at maximum sensitivity.

Oftentimes, not every inhalation/inspiration of a patient is detected to then be classified as an inhalation/inspiration event for the administration of a pulse of gas (e.g., NO). Errors in detection can occur, particularly when multiple gases are being administered to a patient simultaneously, e.g., NO and oxygen combination therapies.

Embodiments of the present invention, and in particular an embodiment which incorporates a breath slope trigger alone or in combination with another trigger, can maximize the correct detection of inspiration events to thereby maximize the effectiveness and efficiency of a therapy while also minimizing waste due to misidentification or errors in timing.

In certain embodiments, greater than 50% of the total number of inspirations of a patient over a timeframe for gas delivery to the patient are detected. In certain embodiments, greater than 75% of the total number of inspirations of a patient are detected. In certain embodiments, greater than 90% of the total number of inspirations of a patient are detected. In certain embodiments, greater than 95% of the total number of inspirations of a patient are detected. In certain embodiments, greater than 98% of the total number of inspirations of a patient are detected. In certain embodiments, greater than 99% of the total number of inspirations of a patient are detected. In certain embodiments, 75% to 100% of the total number of inspirations of a patient are detected.

Dosages and Dosing Regimens

In an embodiment of the invention, nitric oxide delivered to a patient is formulated at concentrations of about 3 to about 18 mg NO per liter, about 6 to about 10 mg per liter, about 3 mg NO per liter, about 6 mg NO per liter, or about 18 mg NO per liter. The NO may be administered alone or in combination with an alternative gas therapy. In certain embodiments, oxygen (e.g., concentrated oxygen) can be administered to a patient in combination with NO.

[0070]    In an embodiment of the present invention, a volume of nitric oxide is administered (e.g., in a single pulse) in an amount of from about 0.350mL to about 7.5mL per breath. In some embodiments, the volume of nitric oxide in each pulse dose may be identical during the course of a single session. In some embodiments, the volume of nitric oxide in some pulse doses may be different during a single timeframe for gas delivery to a patient. In some embodiments, the volume of nitric oxide in each pulse dose may be adjusted during the course of a single timeframe for gas delivery to a patient as breath patterns are monitored. In an embodiment of the invention, the quantity of nitric oxide (in ng) delivered to a patient for purposes of treating or alleviating symptoms of a pulmonary disease on a per pulse basis (the "pulse dose") is calculated as follows and rounded to the nearest nanogram value:

Dose mcg/kg-IBW/hr x Ideal body weight in kg (kg-IBW) x ((1 hr/60 min) x (1 min/respiratory rate (bpm)) x (1,000ng/ug).

As an example, Patient A at a dose of 100 mcg/kg IBW/hr has an ideal body weight of 75kg, has a respiratory rate of 20 breaths per minute (or 1200 breaths per hour):

$$100 \text{ mcg/kg-IBW/hr x } 75 \text{ kg x } (1 \text{ hr}/1200 \text{ breaths}) \text{ X } (1,000 \text{ ng/ug}) = 6250 \text{ ng per pulse}$$

In certain embodiments, the 60/respiratory rate (ms) variable may also be referred to as the Dose Event Time. In another embodiment of the invention, a Dose Event Time is 1 second, 2 seconds, 3 seconds, 4 seconds, 5 seconds, 6 seconds, 7 seconds, 8 seconds, 9 seconds, or 10 seconds.

In an embodiment of the invention, a single pulse dose provides a therapeutic effect (e.g., a therapeutically effective amount of NO) to the patient. In another embodiment of the invention, an aggregate of two or more pulse doses provides a therapeutic effect (e.g., a therapeutically effective amount of NO) to the patient.

In an embodiment of the invention, at least about 300, about 310, about 320, about 330, about 340, about 350, about 360, about 370, about 380, about 390, about 400, about 410, about 420, about 430, about 440, about 450, about 460, about 470, about 480, about 490, about 500, about 510, about 520, about 530, about 540, about 550, about 560, about 570, about 580, about 590, about 600, about 625, about 650, about 675, about 700, about 750, about 800, about 850, about 900, about 950, or about 1000 pulses of nitric oxide is administered to a patient every hour.

In an embodiment of the invention, a nitric oxide therapy session occurs over a timeframe. In one embodiment, the timeframe is at least about 1 hour, about 2 hours, about 3 hours, about 4 hours, about 5 hours, about 6 hours, about 7 hours, about 8 hours, about 9 hours, about 10, hours, about 11 hours, about 12 hours, about 13 hours, about 14 hours, about 14 hours, about 15 hours, about 16 hours, about 17 hours, about 18 hours, or about 24 hours per day.

In an embodiment of the invention, a nitric oxide treatment is administered for a timeframe of a minimum course of treatment. In an embodiment of the invention, the minimum course of treatment is about 10 minutes, about 15 minutes, about 20 minutes, about 30 minutes, about 40 minutes, about 50 minutes, about 60 minutes, about 70 minutes, about 80 minutes, or about 90 minutes. In an embodiment of the invention, the minimum course of treatment is about 1 hour, about 2 hours, about 3 hours, about 4 hours, about 5 hours, about 6 hours, about 7 hours, about 8 hours, about 9 hours, about 10, hours, about 11 hours, about 12 hours, about 13 hours, about 14 hours, about 14 hours, about 15 hours, about 16 hours, about 17 hours, about 18 hours, or about 24 hours. In an embodiment of the invention, the minimum course of treatment is about 1, about 2, about 3, about 4, about 5, about 6, or about 7 days, or about 1, about 2, about 3, about 4, about 5, about 6, about 7, or about 8 weeks, or about 1, about 2, about 3, about 4, about 5, about 6, about 7, about 8, about 9, about 10, about 11, about 12, about 18, or about 24 months.

In an embodiment of the invention, a nitric oxide treatment session is administered one or more times per day. In an embodiment of the invention, nitric oxide treatment session may be once, twice, three times, four times, five times, six times, or more than six times per day. In an embodiment of the invention, the treatment session may be administered once a month, once every two weeks, once a week, once every other day, daily, or multiple times in one day.

Timing of a Pulse of NO

In the invention, the breath pattern is correlated with an algorithm to calculate the timing of administration of a dose of nitric oxide.

The precision of detection of an inhalation/inspiration event also permits the timing of a pulse of gas (e.g., NO) to maximize its efficacy by administering gas at a specified time frame of the total inspiration time of a single detected breath.

In an embodiment of the invention, at least fifty percent (50%) of the pulse dose of a gas is delivered over the first third of the total inspiratory time of each breath. In an embodiment of the invention, at least sixty percent (60%) of the pulse dose of a gas is delivered over the first third of the total inspiratory time. In an embodiment of the invention, at least seventy-five percent (75%) of the pulse dose of a gas is delivered over the first third of the total inspiratory time for each breath. In an embodiment of the invention, at least eighty-five (85%) percent of the pulse dose of a gas is delivered over the first third of the total inspiratory time for each breath. In an embodiment of the invention, at least ninety percent (90%) of the pulse dose of a gas is delivered over the first third of the total inspiratory time. In an embodiment of the invention, at least ninety-two percent (92%) of the pulse dose of a gas is delivered over the first third of the total inspiratory time. In an embodiment of the invention, at least ninety-five percent (95%) of the pulse dose of a gas is delivered over the first third of the total inspiratory time. In an embodiment of the invention, at least ninety-nine (99%) of the pulse dose of a gas is delivered over the first third of the total inspiratory time. In an embodiment of the invention, 90% to 100% of the pulse dose of a gas is delivered over the first third of the total inspiratory time.

In an embodiment of the invention, at least seventy percent (70%) of the pulse dose is delivered to the patient over the first half of the total inspiratory time. In yet another embodiment, at least seventy-five percent (75%) of the pulse dose is delivered to the patient over the first half of the total inspiratory time. In an embodiment of the invention, at least eighty percent (80%) of the pulse dose is delivered to the patient over the first half of the total inspiratory time. In an embodiment of the invention, at least 90 percent (90%) of the pulse dose is delivered to the patient over the first half of the total inspiratory time. In an embodiment of the invention, at least ninety-five percent (95%) of the pulse dose is delivered to the patient over the first half of the total inspiratory time. In an embodiment of the invention, 95% to 100% of the pulse dose of a gas is delivered over the first half of the total inspiratory time

In an embodiment of the invention, at least ninety percent (90%) of the pulse dose is delivered over the first two-thirds of the total inspiratory time. In an embodiment of the invention, at least ninety-five percent (95%) of the pulse dose is delivered over the first two-thirds of the total inspiratory time. In an embodiment of the invention, 95% to 100% of the pulse dose is delivered over the first two-thirds of the total inspiratory time.

When aggregated, administration of a number of pulse doses over a therapy session/timeframe can also meet the above ranges. For example, when aggregated greater than 95% of all the pulse doses administered during a therapy session were administered over the first two thirds of all of the inspiratory times of all of the detected breaths. In higher precision embodiments, when aggregated greater than 95% of all the pulse doses administered during a therapy session were administered over the first third of all of the inspiratory times of all of the detected breaths.

Given the high degree of precision of the detection methodologies of the present invention, a pulse dose can be administered during any specified time window of an inspiration. For example, a pulse dose can be administered targeting the first third, middle third or last third of a patient's inspiration. Alternatively, the first half or second half of an inspiration can be targeted for pulse dose administration. Further, the targets for administration may vary. In one embodiment, the first third of an inspiration time can be targeted for one or a series of inspirations, where the second third or second half may be targeted for one or a series of subsequent inspirations during the same or different therapy session. Alternatively, after the first quarter of an inspiration time has elapsed the pulse dose begins and continues for the middle half (next two quarters) and can be targeted such that the pulse dose ends at the beginning of the last quarter of inspiration time. In some embodiments, the pulse may be delayed by 50, 100, or 200 milliseconds (ms) or a range from about 50 to about 200 milliseconds.

The utilization of a pulsed dose during inhalation reduces the exposure of poorly ventilated areas of the lung and alveoli from exposure to a pulsed dose gas, e.g., NO. In one embodiment, less than 5% of poorly ventilated (a) areas of the lung or (b) alveoli are exposed to NO. In one embodiment, less than 10% of poorly ventilated (a) areas of the lung or (b) alveoli are exposed to NO. In one embodiment, less than 15% of poorly ventilated (a) areas of the lung or (b) alveoli are exposed to NO. In one embodiment, less than 20% of poorly ventilated (a) areas of the lung or (b) alveoli are exposed to NO. In one embodiment, less than 25% of poorly ventilated (a) areas of the lung or (b) alveoli are exposed to NO. In one embodiment, less than 30% of poorly ventilated (a) areas of the lung or (b) alveoli are exposed to NO. In one embodiment, less than 50% of poorly ventilated (a) areas of the lung or (b) alveoli are exposed to NO. In one embodiment, less than 60% of poorly ventilated (a) areas of the lung or (b) alveoli are exposed to NO. In one embodiment, less than 70% of poorly ventilated (a) areas of the lung or (b) alveoli are exposed to NO. In one embodiment, less than 80% of poorly ventilated (a) areas of the lung or (b) alveoli are exposed to NO. In one embodiment, less than 90% of poorly ventilated (a) areas of the lung or (b) alveoli are exposed to NO.

Methods of Treatment

The present invention relates to iNO for use in methods for increasing activity levels in patients with lung-related conditions in accordance with the claims. The iNO is for use in methods of the invention including administration of iNO, optionally supplementing iNO administration with oxygen. The iNO for use in methods of the invention is administered according to the pulsed manner set out in the claims. In an embodiment of the invention, the iNO is delivered to a patient using the INOpulse® device. In one embodiment, the patient is administered iNO for a period of at least about 12 hours, 13 hours, 14, hours, 15 hours, 16 hours, 17 hours, 18 hours, 19 hours, 20 hours, 21 hours, 22 hours, 23 hours, or 24 hours per day for a period of at least about 1 week, 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks or 20 weeks. In one embodiment, the patient is administered iNO for 8 weeks. In another embodiment, the patient is administered iNO for 16 weeks. In an embodiment of the invention, a nitric oxide therapy session occurs over a timeframe. In one embodiment, the timeframe is at least about 1 hour, about 2 hours, about 3 hours, about 4 hours, about 5 hours, about 6 hours, about 7 hours, about 8 hours, about 9 hours, about 10, hours, about 11 hours, about 12 hours, about 13 hours, about 14 hours, about 14 hours, about 15 hours, about 16 hours, about 17 hours, about 18 hours, or about 24 hours per day.

In an embodiment of the invention, a nitric oxide treatment is administered for a timeframe of a minimum course of treatment. In an embodiment of the invention, the minimum course of treatment is about 10 minutes, about 15 minutes, about 20 minutes, about 30 minutes, about 40 minutes, about 50 minutes, about 60 minutes, about 70 minutes, about 80 minutes, or about 90 minutes. In an embodiment of the invention, the minimum course of treatment is about 1 hour, about 2 hours, about 3 hours, about 4 hours, about 5 hours, about 6 hours, about 7 hours, about 8 hours, about 9 hours, about 10, hours, about 11 hours, about 12 hours, about 13 hours, about 14 hours, about 14 hours, about 15 hours, about 16 hours, about 17 hours, about 18 hours, or about 24 hours. In an embodiment of the invention, the minimum course of treatment is about 1, about 2, about 3, about 4, about 5, about 6, or about 7 days, or about 1, about 2, about 3, about 4, about 5, about 6, about 7, or about 8 weeks, or about 1, about 2, about 3, about 4, about 5, about 6, about 7, about 8, about 9, about 10, about 11, about 12, about 18, or about 24 months.

In an embodiment of the invention, the iNO is administered at anywhere from 10 mcg/kg ideal body weight (IBW)/hr to 200 mcg/kg IBW/hr or more. In one embodiment, the iNO is administered from about 20 mcg/kg IBW/hr to about 150 mcg/kg IBW/hr. In one embodiment, the iNO is administered from about 25 mcg/kg IBW/hr to about 100 mcg/kg IBW/hr. In one embodiment, the iNO is administered from about 30 mcg/kg IBW/hr to about 75 mcg/kg IBW/hr. In one embodiment, the iNO is administered from about 25 mcg/kg IBW/hr to about 50 mcg/kg IBW/hr. In one embodiment, the iNO is administered from about 30 mcg/kg IBW/hr to about 45 mcg/kg IBW/hr. In one embodiment, the iNO is administered at 25 mcg/kg IBW/hr. In one embodiment, the iNO is administered at 30 mcg/kg IBW/hr. In one embodiment, the iNO is administered at 35 mcg/kg IBW/hr. In one embodiment, the iNO is administered at 40 mcg/kg IBW/hr. In one embodiment, the iNO is administered at 45 mcg/kg IBW/hr. In one embodiment, the iNO is administered at 50 mcg/kg IBW/hr. In one embodiment, the iNO is administered at 55 mcg/kg IBW/hr. In one embodiment, the iNO is administered at 60 mcg/kg IBW/hr. In one embodiment, the iNO is administered at 65 mcg/kg IBW/ hr. In one embodiment, the iNO is administered at 70 mcg/kg IBW/ hr. In one embodiment, the iNO is administered at 75 mcg/kg IBW/ hr. In one embodiment, the iNO is administered at 80 mcg/kg IBW/ hr. In one embodiment, the iNO is administered at 85 mcg/kg IBW/ hr. In one embodiment, the iNO is administered at 90 mcg/kg IBW/ hr. In one embodiment, the iNO is administered at 95 mcg/kg IBW/ hr. In one embodiment, the iNO is administered at 100 mcg/kg IBW/ hr. In one embodiment, the iNO is administered at 105 mcg/kg IBW/kg. In one embodiment, the iNO is administered at 110 mcg/kg IBW/ hr. In one embodiment, the iNO is administered at 115 mcg/kg IBW/ hr. In one embodiment, the iNO is administered at 120 mcg/kg IBW/ hr. In one embodiment, the iNO is administered at 125 mcg/kg IBW/ hr. In one embodiment, the iNO is administered at 130 mcg/kg IBW/ hr. In one embodiment, the iNO is administered at 135 mcg/kg IBW/ hr. In one embodiment, the iNO is administered at 140 mcg/kg IBW/ hr. In one embodiment, the iNO is administered at 145 mcg/kg IBW/ hr. In one embodiment, the iNO is administered at 150 mcg/kg IBW/ hr. In one embodiment, the iNO is administered at 155 mcg/kg IBW/ hr. In one embodiment, the iNO is administered at 160 mcg/kg IBW/ hr. In one embodiment, the iNO is administered at 165 mcg/kg IBW/ hr. In one embodiment, the iNO is administered at 170 mcg/kg IBW/ hr. In one embodiment, the iNO is administered at 175 mcg/kg IBW/ hr. In one embodiment, the iNO is administered at 180 mcg/kg IBW/ hr. In one embodiment, the iNO is administered at 185 mcg/kg IBW/ hr. In one embodiment, the iNO is administered at 190 mcg/kg IBW/ hr. In one embodiment, the iNO is administered at 195 mcg/kg IBW/ hr. In one embodiment, the iNO is administered at 200 mcg/kg IBW/ hr.

In an embodiment of the invention, the patient is also administered oxygen with the iNO. In an embodiment of the invention, the oxygen is administered at up to 20L/minute. In an embodiment of the invention, the oxygen is administered at up to 1L/ minute, 2L/ minute, 3L/ minute, 4L/ minute, 5L/ minute, 6L/ minute, 7L minute, 8L/ minute, 9L/ minute, 10L/minute, 11L/minute, 12L/minute, 13L/minute, 14L/minute, 15L/minute, 16L/minute, 17L/minute, 18L/minute, 19L/minute, or 20L/minute. In an embodiment of the invention, oxygen is administered as prescribed by a physician.

In an example of the disclosure, the lung related condition is selected from idiopathic pulmonary fibrosis (IPF), pulmonary fibrosis (PF), interstitial lung disease (ILD), pulmonary arterial hypertension (PAH), chronic obstructive pulmonary disorder (COPD), cystic fibrosis (CF), and emphysema. In an example of the disclosure, the pulmonary disease is pulmonary hypertension associated with other pulmonary diseases such as Group I-V pulmonary hypertension (PH). In an embodiment of the invention, the pulmonary disease and/or lung-related condition is pulmonary hypertension associated with interstitial lung disease. In an example of the disclosure, the pulmonary disease and/or lung-related condition is pulmonary hypertension associated with pulmonary fibrosis. In an embodiment of the invention, the pulmonary disease and/or lung-related condition is pulmonary hypertension associated with idiopathic pulmonary fibrosis. In an embodiment of the invention, a patient suffering from ILD is at high risk of developing pulmonary hypertension. In another embodiment of the invention, a patient suffering from ILD is at low risk of developing pulmonary hypertension. In an embodiment of the invention, a patient suffering from ILD is at medium risk of developing pulmonary hypertension. In an embodiment of the invention, a patient suffering from IPF is at high risk of developing pulmonary hypertension. In an embodiment of the invention, a patient suffering from IPF is at medium risk of developing pulmonary hypertension. In another embodiment of the invention, a patient suffering from IPF is at low risk of developing pulmonary hypertension. In an example of the disclosure, a patient suffering from PF is at high risk of developing pulmonary hypertension. In an example of the disclosure, a patient suffering from PF is at medium risk of developing pulmonary hypertension. In an example of the disclosure, a patient suffering from PF is at low risk of developing pulmonary hypertension.

In particular, patients with pulmonary hypertension associated with interstitial lung disease demonstrated a statistically significant improvement in activity levels as measured by actigraphy (wearable, medical-grade activity monitor). Actigraphy is currently being used as the primary endpoint in multiple late-stage clinical programs for pulmonary hypertension and other cardiopulmonary diseases. Patients on iNO demonstrated an increase in moderate activity versus a decrease in patients on placebo. Patients on iNO therapy also did not show any decline in their overall activity levels versus a decline in patients on placebo. The patients also experienced clinical meaningful improvements in other areas as well. NT-ProBNP, which is a peptide marker for right ventricular failure, was measured, and patients on iNO therapy showed only a small increase in NT-ProBNP versus a larger increase for patients on placebo. Higher levels of NT-ProBNP are indicative of a worsening disease state, and these results are consistent with activity results (i.e., showing greater worsening in placebo patients). Also, patients on iNO demonstrated improved oxygen saturation: $SpO_2$ nadir improved in patients on iNO therapy, whereas $SpO_2$ nadir worsened for patients on placebo. In addition, in an embodiment of the invention, oxygen desaturation improved in patients receiving iNO therapy, versus a worsening for patients on placebo. Measured another way, in an embodiment of the invention, oxygen desaturation improved by in patients receiving iNO therapy, versus a worsening for patients on placebo. Unlike other systemic vasodilators, such as those approved for treatment of pulmonary arterial hypertension, INOpulse's targeted delivery to the lungs improves oxygen saturation during exercise.

In an embodiment of the invention, other parameters useful in assessing the effects of iNO include time to clinical improvement and time to clinical worsening. A shortening of the time it takes to see clinical improvement and a lengthening of the time it takes to see clinical worsening is expected in patients treated with iNO. Patient related outcome measurements (PROs) are also useful in assessing the effects of iNO. PROs are measured in the form of questionnaires, which provide a subject's perspective on overall quality of life. In an embodiment of the invention, these PROs include the St. George's Respiratory Questionnaire (SGRQ) and the University of California, San Diego Shortness of Breath Questionnaire (UCSD SOBQ). Both of these Questionnaires are standard questionnaires used in the art and are well-known and clinically accepted. An improvement in scores for both of these PROs is expected for patients on iNO therapy.

Actigraphy

The present invention relates to iNO for use in methods of improving or maintaining activity levels, or preventing a decline in activity levels, in patients with a cardiopulmonary condition or lung-related conditions in accordance with the claims. The methods include using actigraphy to monitor and measure changes in activity level.

Actigraphy involves use of a wearable activity monitor, similar to a pedometer or a accelerometer, or a triaxial accelerometer, an Actigraph GT9X, or a FITBIT®, that measures activity parameters. Such activity monitor assesses activity and measures activity parameters of the user. Activity parameters measured include overall activity, non-sedentary activity, moderate activity, moderate to vigorous physical activity (MVPA), steps, calories, metabolic equivalent units (MET), sleep, heart rate, oxygen saturation, calories burned, and other types of activity parameters.

In an embodiment of the invention, activity levels are monitored and measured constantly over a period of time. In an embodiment of the invention, activity levels are monitored and measured intermittently over a period of time. In one embodiment, activity levels are monitored and measured for a period of at least about 8 hours, 9 hours, 10 hours, 11 hours, 12 hours, 13 hours, 14, hours, 15 hours, 16 hours, 17 hours, 18 hours, 19 hours, 20 hours, 21 hours, 22 hours, 23 hours, or 24 hours per day for a period of at least about 1 week, 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, 21 weeks, 22 weeks, 23 weeks, 24 weeks, 25 weeks, or 26 weeks. In another embodiment, activity levels are monitored and measured for a period of at least about 8 hours, 9 hours, 10 hours, 11 hours, 12 hours, 13 hours, 14, hours, 15 hours, 16 hours, 17 hours, 18 hours, 19 hours, 20 hours, 21 hours, 22 hours, 23 hours, or 24 hours per day for a period of at least about 1 month, 2 months, 3 months, 4 months, 5 months, or 6 months. In another embodiment, activity levels are monitored only during times when the patient is awake. In one embodiment, activity levels are measured in a continuous manner over the entire awake time period. In another embodiment, patients may remove the device for certain activities, thus activity levels are measured in a non-continuous manner over the awake time period. In another embodiment, the awake time period is at least 10 hours. In another embodiment, the awake time period is at least 8 hours. In another embodiment, the awake time period is at least 12 hours. In another embodiment, the awake time period is at least 14 hours.

In an embodiment of the invention, activity levels are improved as compared with a baseline activity level. In an embodiment, the baseline activity level is monitored and measured for at least one week prior to administration of vasodilators. In another embodiment, baseline activity level is monitored or measured for about 1 day to about 14 days, for about 1 day to about 10 days, for about 1 day to about 7 days, or for about 1 day to about 5 days. In another embodiment, baseline activity level is monitored or measured for about 1 day, 2 days, 3 days, 4 days, 5 days, 6 days, 7 days, 8 days, 9 days, 10 days, 11 days, 12 days, 13 days, or 14 days. In an embodiment of the invention, baseline activity is monitored or measured for about 7 days. In an embodiment of the invention, baseline activity level is monitored or measured for a period of about 6 hours, 7 hours, 8 hours, 9 hours, 10 hours, 11 hours, 12 hours, 13 hours, 14 hours 15 hours, 16 hours 17 hours, 18 hours, 19 hours, 20 hours, 21 hours, 22 hours, 23 hours, or 24 hours per day. In an embodiment of the invention, baseline activity is monitored or measured while the subject is awake. In an embodiment of the invention, baseline activity is monitored or measured while the subject is asleep. In an embodiment of the invention, baseline activity is monitored or measured during hours in which the subject is awake and asleep.

In one embodiment, activity levels are improved as compared with a baseline activity level. In one embodiment, activity levels are improved by about 1% to about 50%. In another embodiment, activity levels are improved by about 1% to about 25%. In another embodiment, activity levels are improved by about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, or 25%. In another embodiment, activity levels are improved by about 10%, 15%, 20%, 25%, 30%, 35%, 40%, 45%, or 50%.

In another embodiment of the invention, activity levels are maintained as compared with a baseline activity level. In another embodiment, activity levels do not decrease as compared with a baseline activity level. In another embodiment, activity levels decline less over time in treated patients than untreated or placebo patients. In one embodiment, activity levels decline by about 5% in treated patients, while activity levels decline by about 20% or more for placebo or untreated patients.

In an embodiment of the invention, a subject wears an actigraphy monitor on the non-dominant arm. Wrist acceleration is continually measured by the monitor. The monitor records tri-axial acceleration at 30Hz. An algorithm converts acceleration measurements into minute-by-minute activity counts. Each minute is classified at an activity level based on established and validated cutpoints. Algorithms can also determine wear time, calories, and other parameters. Daily activity data is converted into weekly activity levels to allow data comparison. Predetermined filters are utilized to ensure that only compliant data is analyzed. Such filters may include a minimum number of "wear awake" minutes (e.g., at least 600 minutes) to ensure compliance and having at least three compliant days for a complaint week. Filters may be based on industry standards used for actigraphy analysis.

Counts can be converted into activity levels in multiple ways. For example, the average of the counts provides a direct measure of physical activity. Each minute of the day can be converted into an activity intensity allowing the amount of time in sedentary, light, moderate, and vigorous activities to be determined as shown in Table 1:

### Table 1:  Physical Activity Parameter Examples

| Activity Intensity | Example activities | Average Baseline Levels |
| --- | --- | --- |

| Sedentary (<100 counts) (< 1.5 MET) | Lying Standing Computer work | 450 mins (7.5 hours) |
|---|---|---|
| Light (100 -1951 counts) (1.6 - 3.0 MET) | Washing dishes Washing windows Vacuuming | 491 mins (8.2 hours) |
| Moderate (1952-5724 counts) (3.1 - 6.0 MET) | Walking Ascending/descending stairs Lawn mowing | 63 mins (1.1 hours) |
| Vigorous (>5724 counts) (> 6.0 MET) | Slow/fast running Intense sports | 0 mins |

Cardiopulmonary and Lung-Related Conditions

The method of the present disclosure is useful in patients having a cardiopulmonary condition and/or lung-related conditions, including but not limited to idiopathic pulmonary fibrosis (IPF), pulmonary hypertension, pulmonary arterial hypertension (PAH), including Groups I-V pulmonary hypertension (PH), chronic obstructive pulmonary disorder (COPD), cystic fibrosis (CF), emphysema, asthma, interstitial lung disease, and pulmonary fibrosis.

In an example of the disclosure, the pulmonary disease is pulmonary hypertension associated with other pulmonary diseases such as Group I-V pulmonary hypertension (PH). In an embodiment of the invention, the pulmonary disease and/or lung-related condition is pulmonary hypertension associated with interstitial lung disease. In an example of the disclosure, the pulmonary disease and/or lung-related condition is pulmonary hypertension associated with pulmonary fibrosis. In an embodiment of the invention, the pulmonary disease and/or lung-related condition is pulmonary hypertension associated with idiopathic pulmonary fibrosis. In an embodiment of the invention, a patient suffering from ILD is at high risk of developing pulmonary hypertension. In another embodiment of the invention, a patient suffering from ILD is at low risk of developing pulmonary hypertension. In an embodiment of the invention, a patient suffering from ILD is at medium risk of developing pulmonary hypertension. In an embodiment of the invention, a patient suffering from IPF is at high risk of developing pulmonary hypertension. In an embodiment of the invention, a patient suffering from IPF is at medium risk of developing pulmonary hypertension. In another embodiment of the invention, a patient suffering from IPF is at low risk of developing pulmonary hypertension. In an example of the disclosure, a patient suffering from PF is at high risk of developing pulmonary hypertension. In an example of the disclosure, a patient suffering from PF is at medium risk of developing pulmonary hypertension. In an example of the disclosure, a patient suffering from PF is at low risk of developing pulmonary hypertension.

Other Vasodilators and Administration of Oxygen

The present invention relates to iNO for use in methods of improving or maintaining activity levels, or preventing a decline in activity levels, in patients with a cardiopulmonary condition or lung-related conditions in accordance with the claims. The methods include administering iNO to a patient and then using actigraphy to monitor and measure changes in activity levels.

In an embodiment of the invention, the iNO is administered to the patient in accordance with instructions from a treating physician.

Also described herein are systemic vasodilators and local vasodilators. Systemic vasodilators include, but are not limited to, nitrates. Local vasodilators include, but are not limited to, oxygen, nitric oxide, iNO, sildenafil, tadalafil, and nitroprusside.

In an embodiment of the invention, patients are also administered oxygen with the iNO. In an embodiment of the invention, the oxygen is administered at up to 20L/minute. In an embodiment of the invention, the oxygen is administered at up to 1L/ minute, 2L/ minute, 3L/ minute, 4L/ minute, 5L/ minute, 6L/ minute, 7L minute, 8L/ minute, 9L/ minute, 10L/minute, 11L/minute, 12L/minute, 13L/minute, 14L/minute, 15L/minute, 16L/minute, 17L/minute, 18L/minute, 19L/minute, or 20L/minute. In an embodiment of the invention, oxygen is administered as prescribed by a physician. In another embodiment, the patient is on long-term oxygen therapy (LTOT). In another embodiment, the patient is administered oxygen 24 hours per day. In another embodiment, the patient is administered oxygen for at least 1, 2, 3, 4, 5, 6, 7, 8, 9, 10, 11, 12, 13, 14, 15, 16, 17, 18, 19, 20, 21, 22, 23, or 24 hours per day. In another embodiment, the patient is administered oxygen for at least 12 hours per day.

EXAMPLES

The embodiments encompassed herein are now described with reference to the following examples. These examples are provided for the purpose of illustration only and the disclosure encompassed herein should in no way be construed as being limited to these examples, but rather should be construed to encompass any and all variations which become evident as a result of the teachings provided herein.

Example 1: Determination of Precise Breath Sensitivity for Appropriate Trigger/Arming Thresholds

A device using a threshold algorithm to detect breaths was used in this Example (Device 1). A threshold algorithm detects breaths using pressure; that is a pressure drop below a certain threshold must be met upon inspiration to detect and count a breath. That pressure threshold can be modified as a result of varying the detection sensitivity of the Device 1. Several breath sensitivity settings were tested in the present Example. Settings from 1 to 10 were tested, with 1 being the least sensitive and 10 being the most sensitive. The trigger threshold, shown in cm $H_2O$ is the threshold level at which nitric oxide is delivered. The arming threshold, also shown in cm $H_2O$, is the threshold level at which the device is armed for the next delivery of nitric oxide. The data are shown below in Table 1.

Table 1, below, illustrates a data set collected in this Example. Variation in the breath sensitivity setting resulted in an increase in trigger threshold (measured in cm $H_2O$) from -1.0 at the least sensitive setting (1) to -0.1 at the most sensitive setting (10). In addition, the arming threshold (measured in cm $H_2O$) stayed constant at 0.1 from a sensitivity setting of 1 through a setting of 6, and decreased by 0.02 for each sensitivity setting thereafter through 10. This indicates that the most sensitive breath sensitivity setting allows breaths to be detected more accurately, which leads to more accurate pulsatile delivery of nitric oxide in a shorter window of time, i.e, earlier in the inspiratory part of the breath. Based on these data, additional tests were performed at sensitivity settings of 8 and 10.

**Table 1: Breath Sensitivity and Trigger/Arming Thresholds**

| Breath Sensitivity | 1 | 2 | 3 | 4 | 5 | 6 | 7 | 8 | 9 | 10 |
|---|---|---|---|---|---|---|---|---|---|---|
| Trigger Threshold (cm $H_2O$) | -1.0 | -0.9 | -0.8 | -0.7 | -0.6 | -0.5 | -0.4 | -0.3 | -0.2 | -0.1 |
| Arming Threshold (cm $H_2O$) | +0.1 | +0.1 | +0.1 | +0.1 | +0.1 | +0.1 | +0.08 | +0.06 | +0.04 | +0.02 |

The conclusion is that a higher breath sensitivity setting correlates to a lower trigger threshold and a higher arming threshold, which prepares the device to deliver short, precise pulses of nitric oxide over the therapy treatment course.

Example 2: Testing a Device Against Various Breath Patterns

As discussed above, accurate and timely delivery of nitric oxide is critical to the present invention. In order to ensure that a device will deliver a precise dose of gas within a precise window of time, ten different breath patterns were tested using a mechanical lung and nose model. Ten different simulated breath patterns were analyzed, and the breath patterns had varying respiratory rate (8 to 36 bpm), tidal volume (316 to 912 ml), and Inspiration: Expiration (I/E) ratios (1:1 to 1:4). These variable breath patterns are patterns expected for subjects age 16 and up and are summarized in Table 2. Real world conditions were emulated to the extent possible.

**Table 2: Summary of Breath Patterns Tested**

| Respiratory Rate (bpm) | Male/ Female | Height (cm) | Ideal Body Weight (kg) | Tidal Volume (mL) | Inspiration Time (sec) | I:E Ratio |
|---|---|---|---|---|---|---|
| 8 | F | 174 | 68.1 | 456 | 1.5 | 1:4 |
| 8 | M | 186 | 86.4 | 564 | 1.5 | 1:4 |

(continued)

| Respiratory Rate (bpm) | Male/ Female | Height (cm) | Ideal Body Weight (kg) | Tidal Volume (mL) | Inspiration Time (sec) | I:E Ratio |
|---|---|---|---|---|---|---|
| 12 | F | 152 | 51.9 | 316 | 1.25 | 1:3 |
| 12 | M | 186 | 86.4 | 564 | 1.25 | 1:3 |
| 18 | F | 174 | 68.1 | 456 | 1.1 | 1:2 |
| 18 | M | 186 | 86.4 | 564 | 1.1 | 1:2 |
| 24 | F | 152 | 51.9 | 316 | 1.0 | 1:1.5 |
| 24 | F | 174 | 68.1 | 456 | 1.0 | 1:1.5 |
| 36 | F | 152 | 51.9 | 632 | 0.8 | 1:1 |
| 36 | F | 174 | 68.1 | 912 | 0.8 | 1:1 |

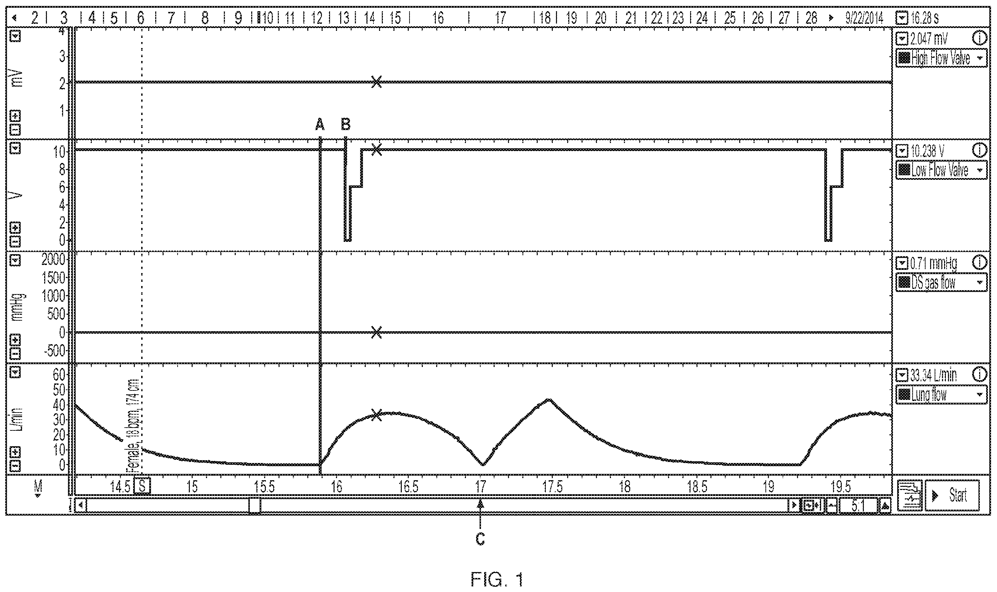

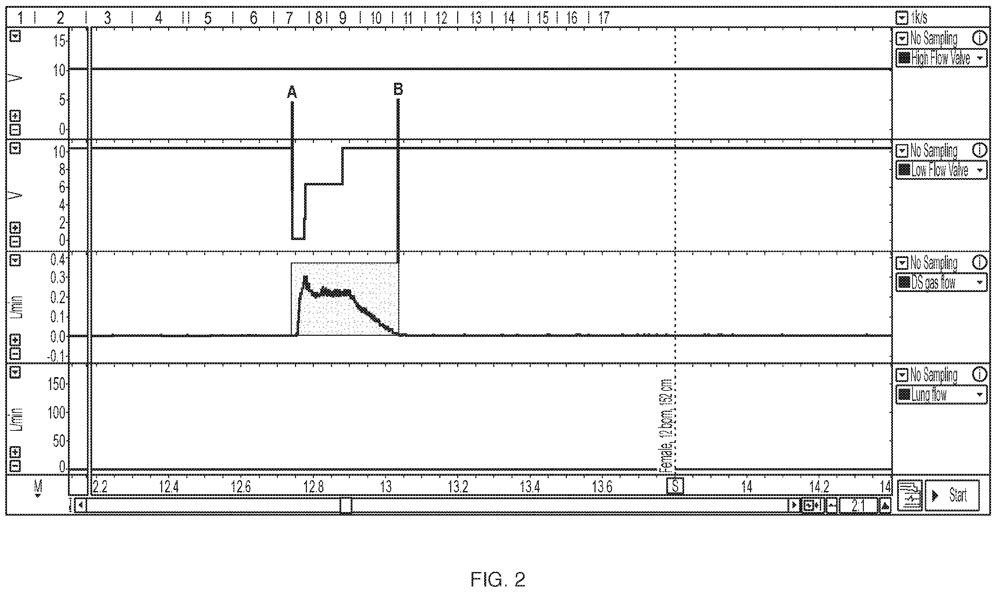

Two devices were tested - Device 1 was tested at sensitivity level 8 and sensitivity level 10, and the other device (Device 2, which further includes a slope algorithm) was tested at sensitivity level 10. The investigation consisted of two parts. Part 1 measured the time delay between the initiation of the inspiratory breath and the onset of nitric oxide delivery using the 10 different simulated respiratory patterns. This time delay is measured using two data points - the time between initiation of inspiration (FIG. 1, Point A) and breath detection with concurrent opening of the delivery valve (FIG. 1, Point B). Part 2 measured the duration and volume of the delivered pulse covering the same breath patterns in Table 2. The time duration of the gas pulse is measured, from breath detection and concurrent opening of the delivery valve, which corresponds to the initiation of gas delivery, (FIG. 2, Point A) to the completion of the gas delivery (FIG. 2, Point B). The volume of the delivered pulse is measured by integration of the gas flow over the pulse duration. In addition, data from Part 1, measured time delay, and Part 2, measured pulse duration, are added to calculate the dose delivery time, sometimes referred to as "delivered pulse width".

Part 1: Measuring time delay between initiation of inspiration and onset of NO delivery. This portion of the test was conducted at a dose of 75 mcg/kg-IBW/hr with a drug concentration input of 6 mg/L (4880 ppm). This test was conducted using nitrogen only. The primary output for Part 1 is time duration between initiation of inspiration and valve opening/breath detection indication. Point A in FIG. 1 is the point where the lung air flow rises just above resting line. The time of valve opening is indicated as Point B in FIG. 1 and is displayed as a sudden voltage drop in the detector. The time interval between Point A and Point B is the valve time delay, or trigger delay, and is calculated for each breath pattern. The total inspiratory time corresponds to the interval from Point A to Point C (which is the end of inspiration).

Part 2: Measuring the duration and volume of the delivered pulse. The same breath patterns were used in this part of the investigation. Doses of 10, 15, 30 and 75 mcg/kg-IBW/hr were tested. The device was programmed for each dose, patient IBW, and respiratory rate (breaths per minute). The resulting pulsatile gas flow was determined by a flow meter. The pulse duration is the time between the point at which the valve opening was indicated, displayed as a sudden voltage drop in the detector, corresponding to Point A in FIG. 2, and the time at which the gas flow returns to baseline at Point B in FIG. 2. The volume of the delivered pulse is the integrated gas flow during the pulse duration. The pulse duration was added to the pulse delay from Part 1 to give the dose delivery time or "delivered pulse width." FIG 1. illustrates the results of Part 1. There are four panels shown in FIG. 1. The second and fourth panels show the breath detection which corresponds to the flow control valve operation and a representation of a breath pattern, respectively. Point A shows initiation of inspiration, Point B shows breath detection which corresponds to the opening of the flow valve, and Point C shows the end of inspiration. From this data, the time delay between points A and B can be calculated.

FIG 2. illustrates the results of Part 2. There are four panels shown in FIG. 2. The second and third panels show the breath detection which corresponds to the flow control valve operation and a representation of the pulsatile gas flow, respectively. Point A shows breath detection which corresponds to the opening of the flow valve and Point B shows the end of pulsatile flow. From this data, the pulse duration between points A and B can be calculated.

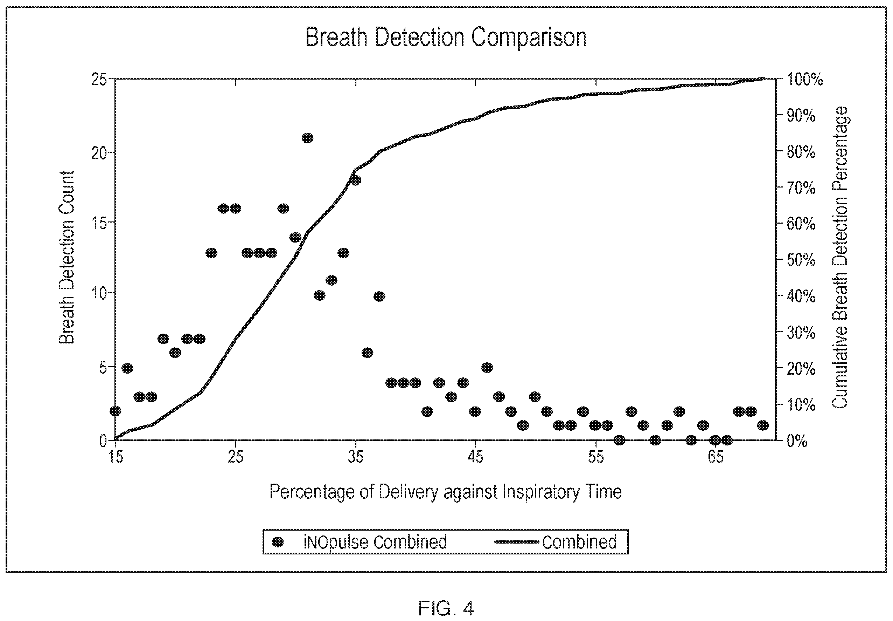

Table 3, below, summarizes the results depicted in FIG. 3 and FIG. 4.

| Device | % Delivery of NO Within Portion of Inspiratory Time | | |
|---|---|---|---|
| | First Third | First Half | First Two-thirds |
| Device 1 (Sensitivity 8) | 17 | 77 | 95 |
| Device 1(Sensitivity 10) | 62 | 98 | 100 |

(continued)

| | % Delivery of NO Within Portion of Inspiratory Time | | |
|---|---|---|---|
| Device | First Third | First Half | First Two-thirds |
| Device 2 | 93 | 100 | 100 |
| Combined Data | 64 | 93 | 99 |

FIG. 3 depicts results for the breath detection count for each device listed in Table 3. The Device 2, or the green data in FIG. 3, illustrates that at least 93% of nitric oxide is delivered within the first third of the inspiratory portion of the breath. 100% of the nitric oxide is delivered within the first half of the inspiratory portion of the breath. Comparatively, for the Device 1 at a sensitivity setting of 8, at least 17% of the nitric oxide is delivered within the first third of the inspiratory portion of the breath, at least 77% within the first half, and at least 95% within the first two-thirds of the inspiratory portion of the breath. The Device 1 at a sensitivity setting of 10 showed results that at least 62% of nitric oxide is delivered within the first third of the inspiratory portion of the breath, at least 98% in the first half, and 100% in the first two-thirds of the inspiratory portion of the breath. FIG. 4 depicts the combined data curve for all three tests.

This data concludes that lower doses of nitric oxide are needed over the course of a single treatment because more nitric oxide is being more precisely delivered with each pulse over a shorter period of time during the course of treatment. Lower doses of nitric oxide may lead to use of less drug overall, and also may lead to less risk of detrimental side effects.

Example 3: Assessment of Activity Parameters in Patients with Pulmonary Hypertension Associated with Interstitial Lung Disease (Cohort 1) - Pulmonary Fibrosis.

Patients were divided into 2 cohorts and randomized 1:1 (Cohort 1) or 2:1 (Cohort 2) for treatment:placebo. Patients were administered either 30 mcg/kg IBW/hr (iNO30, Cohort 1) or 45 mcg/kg IBW/hr (iNO45, Cohort 2) for up to 24 hours per day for a period of either 8 weeks (Cohort 1) or 16 weeks (Cohort 2). Cohort 1 comprised 41 patients and was extended to an open label phase, which is ongoing. The open label phase includes iNO patients dosed with iNO30 or iNO45. The data below represent top-line results for Cohort 1. Cohort 3 will be a pivotal Phase 3 arm using the iNO45 dose.

All patients were administered some level of background oxygen as prescribed by the treating physician for the duration of the study. Vital signs and a baseline 6MWD test were measured on Day 0, and an activity monitor was provided. Patient activity was measured throughout the treatment period using a wearable medical-grade activity monitor (Actigraph GT9X). Vitals and another 6MWD test were measured at Weeks 4 and 8 for cohort 1, and Weeks 12 and 16 for cohort 2.

The Actigraph GT9X is a tri-axial accelerometer that monitors subject movement and acceleration continually at 30 Hz. The acceleration measurements are converted to minute by minute activity "counts." These counts can then be converted into activity intensities based on the established and validated cut-points for each day. Each 1 minute record contains an asleep/awake tag, a wear/non-wear tag, an X, Y, and Z-axis count, and a categorization as sedentary, light, moderate, or vigorous. The minute data is summarized for a daily record containing number of minutes in sedentary, light, moderate, and vigorous activity; cumulative counts (X, Y, and Z axis); number of minutes in non-sedentary activity (sum of light, moderate, and vigorous), number of minutes in VMPA (sum of moderate and vigorous); and overall activity, which is calculated as:

$$\frac{Counts}{minutes} = \frac{\sqrt{(\sum Count_x)^2 + (\sum Count_y)^2 + (\sum Count_z)^2}}{\sum minutes}$$

The monthly and weekly data is calculated by averaging daily data output for all "compliant days" within the period. A compliant day includes ≥600 wear-awake minutes; a compliant month includes ≥14 compliant days within the period; a compliant week includes ≥3 compliant days within the period. Non-compliant days, weeks, or months, were not used in the analysis.

Activities that are meaningful to the pulmonary fibrosis population and relevant to this particular study were identified by and reported within the IPF Voice of Patient - FDA Meeting and Report, 2015. These activities can be grouped by activity intensity based on their estimated metabolic equivalent levels, as summarized below in Table 5A.

Results for cohort 1 are provided here. Results for the six-minute walk distance (6MWD) test support actigraphy. In cohort 1, there were three times more 6MWD improvers in the treatment arm versus placebo. Improvement in 6MWD is defined as greater than or equal to 15% improvement in the 6MWD over the patient's baseline level. Thirty-one percent of patients on treatment improved in 6MWD versus about 11% on placebo, and patients with more severe pulmonary hypertension showed greater improvement over their baseline levels (a +6 meters change for INOpulse patients versus a -7 meters change on placebo). Further, in cohort 1, patients on at least 12 hours of INOpulse resulted in an overall change of +10 meters on the 6MWD versus a -6 meters change on placebo. Composite endpoints of distance saturation product (DSP) and integral DSP (IDSP) are increased compared to 6MWD, which verifies ventilation/perfusion (V/Q) benefits. In addition, iNO was well-tolerated with no safety concerns.

In addition to certain actigraphy parameters (MVPA and Overall Activity), the additional efficacy parameter of NT-proBNP change was also analyzed. NT-proBNP is a natriuretic peptide released by the cardiac myocyte when the ventricle is placed under increased load and stretch (which is caused by pulmonary hypertension). Thus, pulmonary hypertension results in elevated levels of NT-proBNP. Treatment targeting the pulmonary vasculature would be expected to maintain or reduce elevated levels of NT-proBNP. Generally, the higher the baseline level, the greater the potential for reduction.

$SpO_2$ nadir (or lowest oxygen saturation point) during the 6MWT was also measured.

### Table 4: Summary of Key iNO Cohort 1 Outputs

| Parameter | iNO | Placebo | Outcome |
|---|---|---|---|
| Actigraphy (% change)<br>• Moderate Activity<br>• Overall Activity | +8%<br><br>+0% | -26%<br><br>-12% | Statistically significant reduction in moderate activity for placebo (p=0.04)<br>Statistically significant reduction in overall activity for placebo (p=0.05) |
| NT-ProBNP (% change) | +15% | +42% | Peptide marker indicator of cardiac failure<br>Larger increase in placebo indicative of disease worsening |
| Oxygen Saturation<br>• $SpO_2$ Nadir<br>• Oxygen Desaturation | +0.3%<br>-9% | -1.4%<br>+11% | Higher nadir for iNO means better saturation<br>Lower desaturation for iNO means better saturation |

### Table 5: Additional Assessment of Activity Parameters for Cohort 1

| Activity Level | iNO | Placebo | Placebo Corrected Change |
|---|---|---|---|
| Moderate (walking, stairs, yard work, etc.)<br>• All<br>• ≥12 hrs/day on INOpulse | <br>+8%<br>+23% | <br>-26%<br>-30% | <br>+34%<br>+53% |
| Overall Activity (non-sedentary + sedentary)<br>• All<br>• ≥12 hrs/day on INOpulse | <br>0%<br>+2% | <br>-12%<br>-15% | <br>+12%<br>+17% |
| Non-Sedentary (moderate + light activity, e.g., basic housework)<br>• All<br>• ≥12 hrs/day on INOpulse | <br>-6%<br>-3% | <br>-11%<br>-13% | <br>+5%<br>+10% |

### Table 5A: Relevant Physical Activity Metrics

| Voice of Patient Activities | Metabolic Equivalents (MET) | Activity Intensity | | | |
|---|---|---|---|---|---|
| | | Sedentary <1.5 MET | Light 1.6-3.0 MET | Moderate 3.1-6.0 MET | Vigorous >6.1 MET |
| Walking | 2.0-5.0 | | X | X | |
| Climbing Stairs | 3.5-6.0 | | | X | |
| Personal Care (dressing, showering, etc.) | 1.5-2.5 | X | X | | |
| Household Chores | 1.8-6.5 | X | X | X | |
| Exercise and Hobbies (horseback riding, photography, light to moderate exercise) | 1.8-6.0 | | X | X | |

(continued)

| Voice of Patient Activities | Metabolic Equivalents (MET) | Activity Intensity | | | |
|---|---|---|---|---|---|
| | | Sedentary <1.5 MET | Light 1.6-3.0 MET | Moderate 3.1-6.0 MET | Vigorous >6.1 MET |
| Socializing | 1.3-3.0 | X | X | | |

Tables 4 and 5 illustrate changes in key outputs and activity parameters in treated and placebo patients from Cohort 1. Table 4 shows statistically significant data for improvement of activity levels in treated vs. non treated patients. Table 5 shows data for treated and placebo patients, along with the placebo corrected change.

Table 6 illustrates the subject demographics and disease characteristics.

## Table 6:   Patient Demographics and Disease Characteristics

| | iNO 30 (n = 23) | Placebo (n = 18) | Total (n = 41) |
|---|---|---|---|
| Males, n (%) | 16 (69.6) | 13 (72.2) | 29 (70.7) |
| Age (mean, SD) | 68.6 (6.45) | 65.8 (13.73) | 67.4 (10.24) |
| IPF, n (%) | 20 (87.0) | 10 (55.6) | 30 (73.2) |
| Intermediate to High Probability of PH, n (%) | 15 (65.2) | 14 (77.8) | 29 (70.7) |
| DLCO - % predicted (mean, SD) | 30.7 (11.4) | 30.4 (10.2) | 30.5 (10.8) |
| Baseline FVC (mean, L) | 2.2 | 2.3 | 2.2 |
| Baseline FVC (mean, % predicted of normal) | 56.3 (10.2) | 59.9 (18.4) | 57.9 (14.3) |
| Baseline 6MWD – meters (mean, SD) | 293.8 (87.9) | 271.4 (91.3) | 284.0 (89.0) |
| Baseline NT-proBNP – pmol/L (mean, SD) | 17.0 (20.8) | 60.4 (87.7) | 35.5 (62.2) |

Results show iNO provides clinically and statistically significant improvements in activity as measured by a wearable activity monitor (Actigraph GT9X). Changes in NT-ProBNP are consistent with activity results, showing greater worsening for placebo patients. Unlike other approved PAH systemic vasodilators, INOpulse's targeted delivery improves oxygen saturation during exercise. Multiple actigraphy parameters were used to show consistent benefit for subjects on iNO30 vs. placebo. A statistically significant benefit is seen in moderate and overall activity and calories. See a summary of results in Tables 7-10, below.

### Table 7: Summary of iNO-PF Cohort 1 - Actigraphy

| | iNO | Placebo | Placebo Corrected Change |
|---|---|---|---|
| MVPA (min/day) | +8.1% | -26.1% | +43.2% |
| Proportion of awake time in MVPA | +15.8% | -22.0% | +37.8% |

(continued)

|  | iNO | Placebo | Placebo Corrected Change |
|---|---|---|---|
| Overall Activity (counts/min) | +0.0 | -11.9% | -11.9% |
| Calories | -6.1% | -18.1% | +12.0% |

Table 7 shows actigraphy results from the iNO-PF Cohort 1. Improvement in the iNO group was statistically significant as compared with placebo for all parameters: MVPA (p=0.04), proportion of awake time in MVPA (p=0.04), overall activity (p=0.05) and calories (p=0.05). MVPA is a primary endpoint for the pivotal Phase 3 cohort for this trial.

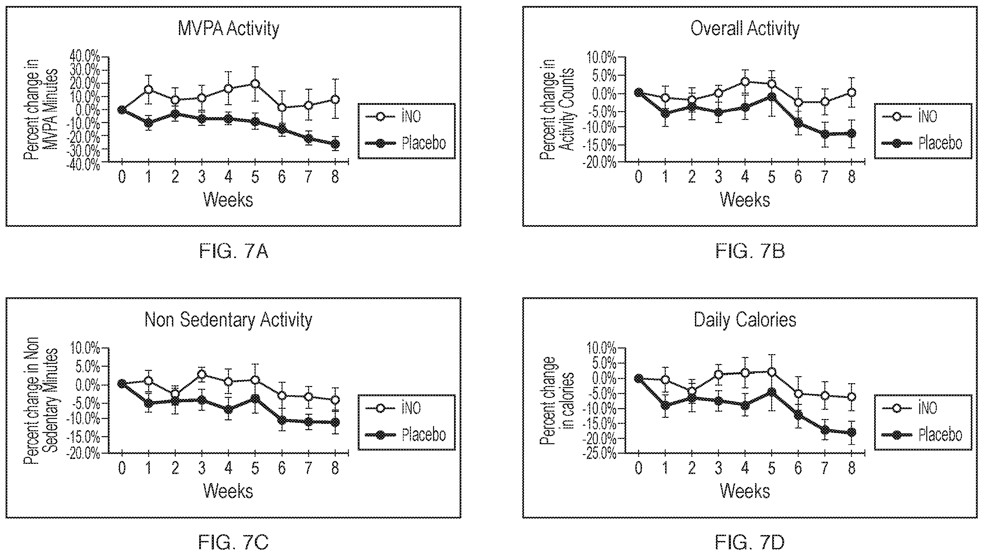

Figure 7A-7D illustrate that iNO demonstrated consistent and sustained benefit in activity parameters of MVPA, overall activity, non-sedentary activity, and daily calories. The data show a consistent split around 4 weeks that is maintained throughout the remainder of the study (through week 8).

**Table 8: Summary of Supporting Results from iNO-PF Cohort 1**

|  | iNO | Placebo | Placebo Corrected Change |
|---|---|---|---|
| Oxygen Desaturation (% improvement) | +9.3% | -10.5% | +19.8% |
| SpO$_2$ Nadir | +0.3% | -1.4% | +1.7% |
| NT-ProBNP (% change) | +15.6% | +42.9% | -27.3% |
| 6MWD | +7.2 m | +0.5 m | +6.7 m |
| Distance Saturation Product (DSP) | +8.5 m% | -2.0 m% | +10.5 m% |

Table 8 shows improvement in supportive parameters. Reduced desaturation and increased SpO$_2$ nadir for the iNO group translates to improved oxygen saturation for that group. The peptide marker NT-ProBNP, which is an indicator for cardiac failure, showed a larger increase in the placebo group, indicative of a worsening disease state.

**Table 9: Safety Data - Cohort 1**

|  | iNO30 (N=23) | Placebo (N=18) |
|---|---|---|
| Adverse Events (AE) | 15 (65.2%) | 13 (72.2%) |
| Serious AE (SAE) | 2 (8.7%) | 2 (11.1%) |
| Deaths | 1 (4.3%) | 0 |
| Discontinuations | 2 (8.7%) | 2 (11.1%) |

Table 9 shows a summary of the safety data. The safety study for Cohort 1 supports a dose escalation to iNO45. There were no serious, unexpected suspected adverse reactions, and there were no unexpected adverse events. All serious adverse events were reported as unrelated to the study. Incidence of AEs and SAEs were low and balanced across both treatment and placebo groups. Pulsed, inhaled nitric oxide is safe and well-tolerated at iNO30.

**Table 10: Change in MVPA**

| Change from Baseline | iNO30 | Placebo |
|---|---|---|
| > +15% | 23% | 0% |
| 0% to +15% | 23% | 14% |
| 0% to -15% | 15% | 14% |
| < -15% | 39% | 71% |

Table 10 shows iNO30 treatment maintains MVPA as compared to a consistent decline in the placebo group. A change in MVPA of 15% or more is considered a significant change. About 23% of patients on iNO showed significant improvement in MVPA, while 71% of placebo patients showed a significant decline in MVPA.

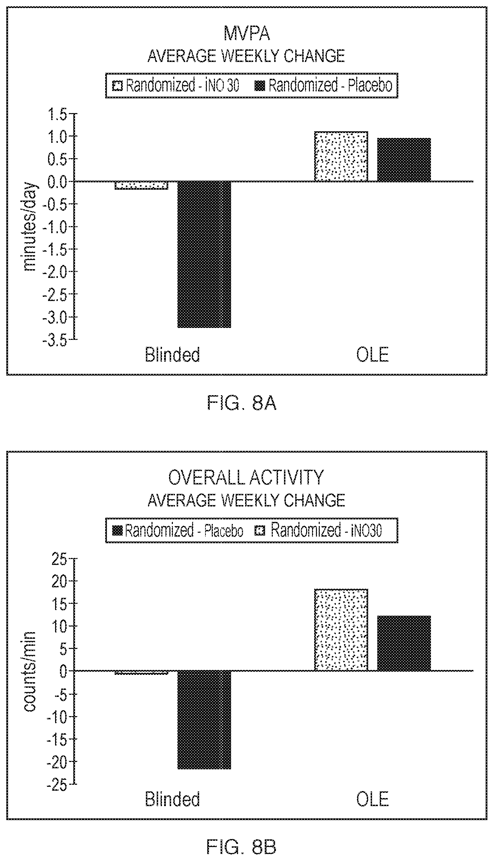

Finally, in Figures 8A and 8B demonstrate consistent improvement for 16 subjects on open label treatment. Figure 8A shows the average weekly change for MVPA in minutes per day and Figure 8B shows the average weekly change in overall activity in counts per minute in treated and placebo patients in both blinded (left panel) and open label extension studies (right panel). Blinded iNO patients showed almost no change in either MVPA or overall activity, whereas blinded placebo patients showed a decrease in MVPA of about 3 minutes and a decrease in overall activity of about 20 counts per minute. On the contrary, in the open label extension (OLE) study, both the OLE iNO and OLE placebo groups showed consistent improvement in both MVPA and overall activity. The OLE iNO group and OLE placebo group showed an improvement of about 1 minute in MVPA, and improvement of about 20 counts per minute and 15 counts per minute, respectively for overall activity.

In summary, a consistent benefit is seen in multiple activity parameters and other supportive parameters for subjects on iNO30 as compared to placebo. MVPA showed the greatest benefit, with a statistically significant placebo corrected benefit of about 34%. Overall activity also showed a statistically significant placebo corrected benefit of about 12%. Other parameters such as non-sedentary activity and calories were supportive of an overall activity benefit for subjects on iNO. Improvements in oxygen saturation and NT-ProBNP are supportive of the INOpulse dual mechanism of action (localized vasodilation and V/Q matching). DSP shows a greater benefit than 6MWD alone, and is consistent with INOpulse's ability to maintain oxygen saturation during exercise. The clinical study is ongoing.

The data shown for Cohort 1, above, is raw data collected during the clinical study for Cohort 1, and does not illustrate a longitudinal analysis. Data shown in Example 4 for both Cohorts 1 and 2 include data analyzed using the Mixed Effect Model Repeated Measure (MMRM) model, and includes pooled placebo data as well.

Example 4: Assessment of Activity Parameters in Patients with Pulmonary Hypertension Associated with Interstitial Lung Disease (Cohort 2) - Pulmonary Fibrosis.

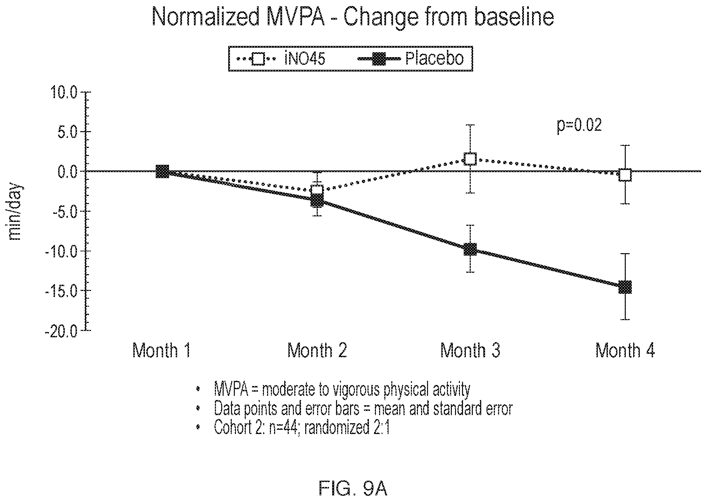

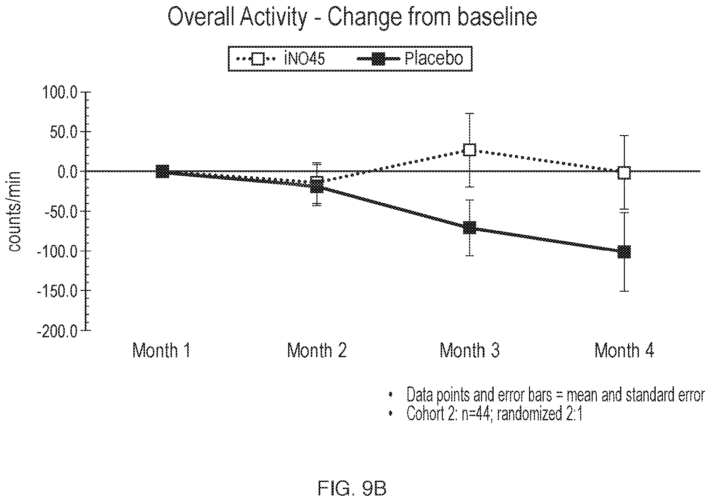

Example 4 illustrates the data collected for Cohort 2 in Example 3, patients dosed with iNO45 (45 mcg/kg IBW/hr), and also includes MMRM analysis relating to Cohorts 1 and 2. Cohort 2 included 44 subjects randomized 2:1 to either iNO45 or placebo for a 4 month (16 weeks) blinded treatment period followed by an open-label extension. Patients from Cohort 2 demonstrated statistically significant improvement in moderate to vigorous physical activity (MVPA), defined as walking, climbing stairs, yard, work, and similar activities, versus placebo. FIG. 9A shows that MVPA is improved by 14 minutes per day, or 20% overall improvement (p=0.02) with treatment with iNO45. FIG. 9B shows that overall activity is improved by 100 counts per minute, representing a 7% improvement.

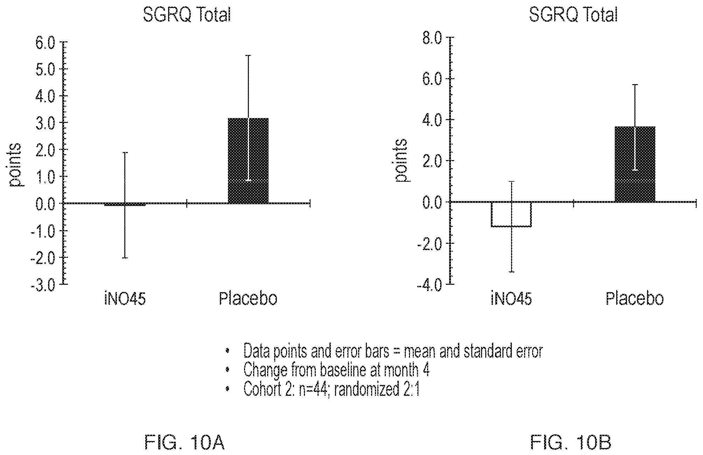

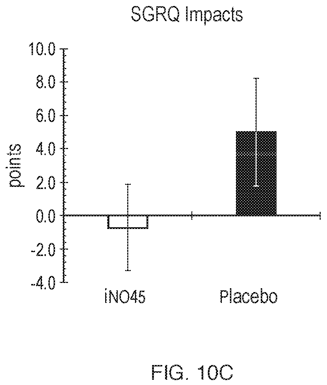

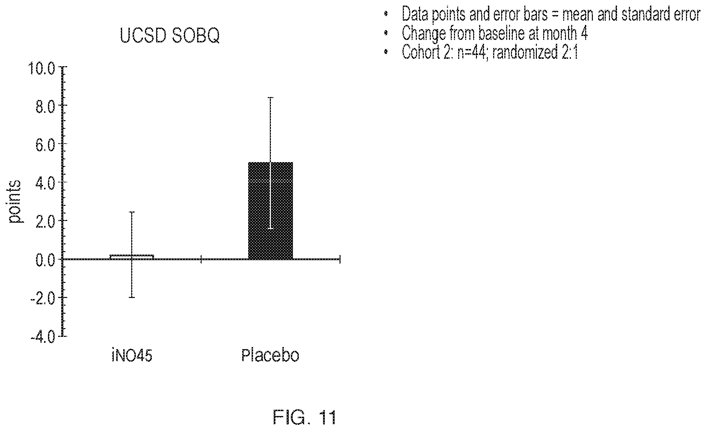

The improvements in actigraphy were directionally supported by improvements in two clinically relevant patient reported outcome measures (PROs), which provide a subject's perspective on overall quality of life and dyspnea or shortness of breath. These PROs include the St. George's Respiratory Questionnaire (SGRQ) and the University of California, San Diego Shortness of Breath Questionnaire (UCSD SOBQ). Both of these Questionnaires are standard questionnaires used in the art and are well-known and clinically accepted. FIGS. 10A-10C illustrate patient scores on the SGRQ. These data demonstrate that patients felt better overall on the iNO45 treatment over placebo. FIG. 11 illustrates patient scores on the UCSD SOBQ, which shows that patients felt less shortness of breath while on the iNO45 treatment over placebo.

Table 11 shows the safety data for Cohort 2 (iNO45). Pulsed iNO was well-tolerated in Cohort 2. Incidence of adverse events (AEs) and serious adverse events (SAEs) was low in both active and placebo groups. AEs were generally non-serious with no observable trends. All SAEs were reported as unrelated to the study drug.

Table 11: Safety Summary: Cohort 2 (iNO45)

|  | **iNO45 (N=30)** | **Placebo (N=14)** |
|---|---|---|
| Adverse Events (AE) | 26 (0.87/subject, 87%) | 9 (0.64/subject, 64%) |
| SAE Reported | 5 (0.17/subject, 17%) | 7 (0.50/subject, 50%) |
| Subjects with SAE | 3 (10%) | 3 (21.4%) |
| Deaths | 0 | 0 |
| Discontinuations | 2 (6.67%) | 0 |

Table 12 illustrates a summary of patient demographics for Cohort 2.

## Table 12: Patient Demographics and Disease Characteristics

| | iNO45 (n = 30) | Placebo (n = 14) | Total (n = 44) |
|---|---|---|---|
| Males (n, %) | 15 (50.0) | 10 (71.4) | 26 (56.8) |
| Age (mean, SD) | 68.9 (9.95) | 71.4 (5.14) | 69.5 (8.75) |
| IPF (n, %) | 19 (63.3) | 12 (85.7) | 31 (70.5) |
| Intermediate to High Probability of PH, n (%) | 18 (60) | 9 (64.3) | 27 (61.4) |
| Baseline DLCO - % predicted (mean, SD) | 35.7 (14.2) | 35.3 (7.9) | 35.6 (12.4) |
| Baseline FVC - % predicted (mean, SD) | 59.7 (15.9) | 60.5 (15.1) | 60.0 (15.5) |
| Baseline 6MWD – meters (mean, SD) | 272.9 (81.3) | 278.7 (76.3) | 274.8 (78.8) |
| Baseline NT-proBNP – pmol/L (mean, SD) | 47.6 (69.1) | 23.2 (18.4) | 40.2 (59.4) |

Table 13 shows the data related to patient compliance for INOpulse administration for Cohorts 1 and 2. Patient compliance was similar for each of Cohorts 1 and 2, and both groups exceeded the target 12 hours per day on average.

### Table 13: INOpulse Compliance

| | Cohort 1 | | | Cohort 2 | | |
|---|---|---|---|---|---|---|
| | iNO30 | Placebo | Total | iNO45 | Placebo | Total |
| Hrs/day (mean, SD) | 13.0 (5.2) | 14.1 (4.8) | 13.5 (5.1) | 12.9 (4.4) | 14.3 (4.3) | 13.4 (4.4) |

Table 14 illustrates patient compliance with wearing the activity monitor for at least 10 hours per day while awake to ensure an accurate assessment of their activity levels during that day. As noted in the table, both cohorts demonstrated average compliance above the required 10 hours per day, but Cohort 2 increased the percent of compliant days from 79% to 88%, indicating improved training in Cohort 2.

### Table 14: Actigraphy Compliance - Wear Awake Time

| | Cohort 1 | | | Cohort 2 | | |
|---|---|---|---|---|---|---|
| | iNO30 | Placebo | Total | iNO45 | Placebo | Total |
| Hrs/day (mean, SD) | 13.0 (7.1) | 15.0 (6.9) | 13.9 (7.1) | 15.0 (5.8) | 16.3 (4.1) | 15.4 (5.4) |
| % Compliant days (≥600 minutes) | 76 | 82 | 79 | 85 | 94 | 88 |

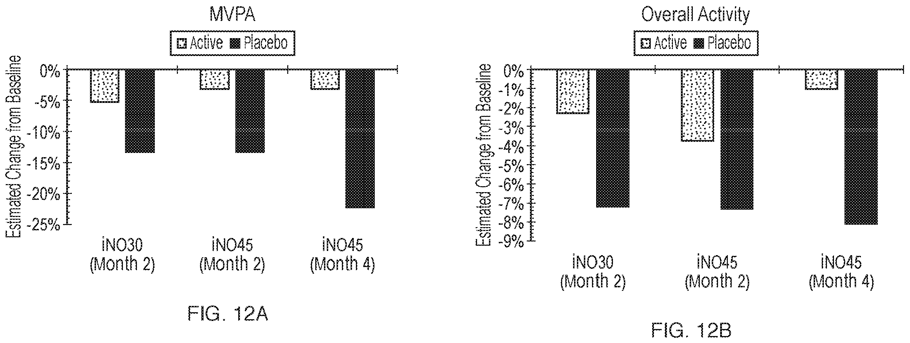

FIG. 12 illustrates the log transformed estimated change in MVPA (FIG. 12A) and Overall Activity (FIG. 12B) at Month 2 (for iNO30 and iNO45) and Month 4 (for iNO45). The Month 2 data is based on pooled placebo data at weeks 4-8, and the Month 4 data is based on data collected between Months 2 and 4. As noted in FIG. 12A, the change from baseline MVPA is smaller for Cohort 2 as compared with Cohort 1. Further, as shown in FIG. 12B, the change from baseline in Overall Activity for Cohort 2 is smaller at 4 months as compared with Cohorts 1 and 2 at 2 months.

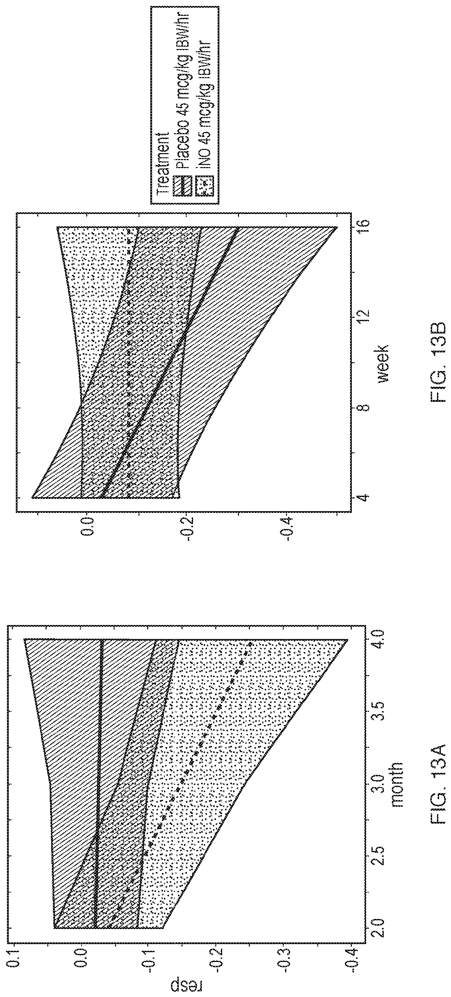

FIG. 13 illustrates the log transformed MVPA predicted marginal effects on a monthly (FIG. 13A) and weekly (FIG. 13B) basis for Cohort 2. In both cases, patients on treatment with iNO45 maintain their activity level while subjects on placebo deteriorate over time. Importantly, the treatment effect is "late" with the difference between the two arms initiating in the 2nd month of treatment and appearing most prominently in the second half of the study. While the shape of the declines is similar between monthly and weekly analyses, the monthly analysis has lower variability with a standard deviation of the residuals of 0.14 as compare to 0.20 in the weekly analysis.

Table 15 illustrates SpO$_2$ nadir during the 6MWD showed minimal difference between the treatment and placebo groups for both Cohorts 1 and 2. The ability to maintain oxygen saturation is consistent with previous results which have shown pulsed iNO can target the well-ventilated alveoli and maintain V/Q (ventilation/perfusion matching) in this patient population.

**Table 15: SpO$_2$ Nadir at Last Analysis Visit**

| | | Difference Between Treatment Groups | | |
|---|---|---|---|---|
| Group | LS Mean (SE) | Estimate | 95% Confidence Interval (CI) | p-value |
| Cohort 1 (wk 8) iNO30 Pooled Placebo | 0.24 (1.00) 1.49 (1.09) | -1.24 | (-4.06, 1.57) | 0.3705 |
| Cohort 2 (wk 8) iNO45 Pooled Placebo | 0.30 (1.13) 1.49 (1.09) | -1.19 | (-4.19, 1.82) | 0.4310 |
| Cohort 2 (wk 16) iNO45 Pooled Placebo | -1.36 (0.96) 0.14 (1.50) | -1.50 | (-5.04, 2.04) | 0.3819 |

Change in NT-proBNP levels were analyzed for Cohorts 1 and 2. In Cohort 1, there was no difference in the change from baseline between the iNO30 and placebo groups as analyzed using the MMRM model. Further, there were no statistically significant differences observed between the iNO30 group in Cohort 1 and pooled placebo using the MMMRM model through week 8. There was a statistically significant difference between iNO45 and pooled placebo through week 8; however, this difference was lost when comparing iNO45 through 16 weeks, possibly due to a smaller sample size. Table 16 illustrates the change in NT-proBNP (pmol/L) through 8 weeks for Cohorts 1 and 2, and through 16 weeks for Cohort 2.

| | | Difference Between Treatment Groups | | |
|---|---|---|---|---|
| Group | LS Mean (SE) | Estimate | 95% Confidence Interval (CI) | p-value |
| Cohort 1 (wk 8) iNO30 Pooled Placebo | -2.53 (1.48) 11.36 (9.53) | -13.90 | (-33.62, 5.83) | 0.1592 |
| Cohort 2 (wk 8) iNO45 Pooled Placebo | -14.98 (7.96) 11.36 (9.53) | -26.34 | (-51.22, -1.47) | 0.0384 |
| Cohort 2 (wk 16) iNO45 Pooled Placebo | 5.29 (9.44) 1.84 (12.35) | 3.45 | (-28.24, 35.13) | 0.8247 |

Time to clinical improvement was a composite endpoint consisting of a ≥15% improvement in 6MWD, or improvement in the SGRQ defined as a reduction of ≥4 points, or ≥15% improvement in DSP/IDSP. There was minimal difference between Cohort 1 and pooled placebo through 8 weeks. Although not statistically significant, Cohort 2 showed a trend towards an increase in the percentage of subjects achieving clinical improvement for iNO45 that was increased when assessed against the larger pooled placebo group. This effect was predominantly driven by an improvement in the SGRQ, further supporting the benefit seen in the overall populations for SGRQ.

Time to clinical worsening was also a composite endpoint consisting of mortality, or hospitalization due to cardiopulmonary worsening, or reduction from baseline in the 6MWD by ≥15%, or worsening functional class. If there were multiple events only the first clinical worsening event was counted. There was minimal difference in the time to clinical worsening for Cohort 1 or Cohort 2, however, iNO45 assessed against the larger pooled placebo showed a trend towards decreased clinical worsening for iNO45, supporting a potential signal which might become more evident in a larger trial.

In summary, a consistent benefit is seen in multiple activity parameters and other supportive parameters for subjects on iNO45 as compared to placebo. MVPA showed the greatest benefit, with a statistically significant placebo corrected benefit of about 20%. Overall activity also showed a statistically significant placebo corrected benefit of about 7%.

Top-line results show that iNO45 provides a clear benefit over placebo in MVPA (moderate to vigorous physical activity) and overall activity over a 4 month treatment period. Subjects on iNO45 maintained their activity levels, whereas subjects on placebo showed a consistent decline. Over 4 months, subjects on iNO45 demonstrated 19% placebo corrected benefit in MVPA supported by a 7% placebo corrected benefit in overall activity. Comparison of iNO30 and iNO45 showed both doses generally maintained activity levels with a potential benefit for iNO45 over iNO30 over 2 months. Due to iNO30 being tested only for 2 months, comparative assessment between the doses at 4 months was not available.

There was a statistically significant improvement in NT-proBNP when comparing iNO45 in Cohort 2 to the pooled placebo group through 8 weeks; however, this effect was lost when iNO45 was compared to placebo through 16 weeks in Cohort 2. The effect at 16 weeks may be due to the smaller sample size and the low number of subjects with abnormal NT-proBNP values at baseline. Oxygen saturation was maintained for both iNO30 and iNO45, consistent with the overall INOpulse mechanism of action of providing pulmonary vasodilation without causing V/Q mismatch.

On PROs, subjects in Cohort 2 generally maintained their SGRQ scores, while subjects on placebo deteriorated. By comparison, subjects in Cohort 1 demonstrated minimal benefit compared to placebo in the SGRQ measures.

Pulsed iNO was well tolerated at iNO30 dose in Cohort 1 and iNO45 dose in Cohort 2 during the blinded treatment period. The AEs were balanced across treatment groups and the incidence of SAEs was low in both Active and Placebo groups in Cohort 1. For Cohort 2, the number of subjects that reported SAEs was lower in the treatment group (10%) as compared to the placebo group (21.4%). Additionally, all SAEs that occurred in both Cohort 1 and 2 during the blinded treatment period, were reported to be unrelated to the drug. There were no unexpected AEs or suspected unexpected serious adverse reactions (SUSARs) that were reported and the benefit/risk profile remains favorable. The consistency of the benefit seen with iNO45 dose, along with the clean safety profile, support progressing this dose into the confirmatory pivotal Phase 3 Cohort, which will assess iNO45 against placebo for a blinded treatment period of 4 months (16 weeks).

While preferred embodiments of the invention are shown and described herein, such embodiments are provided by way of example only and are not intended to otherwise limit the scope of the invention. Various alternatives to the described embodiments of the invention may be employed in practicing the invention.

## Claims

1. Inhaled nitric oxide (iNO) for use in a method for improving or maintaining activity levels in a patient in need thereof, wherein the method comprises administering iNO to the patient, wherein the patient has interstitial lung disease (ILD), wherein the iNO is administered to the patient by:

    (a) detecting a breath pattern in the patient including a total inspiratory time;
    (b) correlating the breath pattern with an algorithm to calculate a timing of administration of a dose of nitric oxide; and
    (c) administering the dose of nitric oxide to the patient in a pulsatile manner over a portion of the total inspiratory time.

2. The iNO for use of claim 1, wherein the inhaled nitric oxide is administered at a dose in a range of from 25 micrograms (mcg)/kg ideal body weight (IBW)/hr to 50 mcg/kg IBW/hr.

3. The iNO for use of claim 2, wherein the inhaled nitric oxide is administered at a dose in a range of from 30 mcg/kg IBW/hr to 45 mcg/kg IBW/hr.

4. The iNO for use of claim 2, wherein the inhaled nitric oxide is administered at a dose of 30 mcg/kg IBW/hr.

5. The iNO for use of claim 2, wherein the inhaled nitric oxide is administered at a dose of 45 mcg/kg IBW/hr.

6. The iNO for use of any one of claims 1-5, wherein activity levels are improved or maintained as compared with a baseline activity level, wherein activity levels comprise one or more activity parameters selected from overall activity, non-sedentary activity, moderate activity, moderate to vigorous physical activity (MVPA), steps, calories, metabolic equivalent units (MET), sleep, heart rate, oxygen saturation, and calories burned.

7. The iNO for use of claim 6, further comprising measuring changes in activity levels using actigraphy.

8. The iNO for use of any one of claims 1-7, wherein the algorithm uses one or both of a threshold sensitivity and a slope algorithm, wherein the slope algorithm detects a breath when the rate of pressure drop reaches a predetermined threshold.

9. The iNO for use of any one of claims 1-8, wherein the interstitial lung disease (ILD) comprises one or more subtypes selected from idiopathic interstitial pneumonia (IIP), chronic hypersensitivity pneumonia, occupational or environmental lung disease, idiopathic pulmonary fibrosis (IPF), non-IPF IIPs, granulomatous, and connective tissue disease related ILD.

10. The iNO for use of any one of claims 1-8, wherein the interstitial lung disease comprises idiopathic pulmonary fibrosis (IPF).

11. The iNO for use of any one of claims 1-10, wherein the patient is at high risk of developing pulmonary hypertension.

**Patentansprüche**

1. Inhalierbares Stickstoffmonoxid (iNO) zur Verwendung in einem Verfahren zur Verbesserung oder Aufrechterhaltung des Aktivitätsniveaus bei einem Patienten, der dies benötigt, wobei das Verfahren die Verabreichung von iNO an den Patienten umfasst, wobei der Patient an einer interstitiellen Lungenerkrankung (ILD) leidet, wobei das iNO dem Patienten verabreicht wird durch:

   (a) Erfassen eines Atemmusters des Patienten einschließlich einer gesamten Inspirationszeit;
   (b) Korrelieren des Atemmusters mit einem Algorithmus, um einen Zeitpunkt für die Verabreichung einer Dosis Stickstoffmonoxid zu berechnen; und
   (c) Verabreichen der Dosis Stickstoffmonoxid an den Patienten in pulsierender Weise über einen Teil der gesamten Inspirationszeit.

2. iNO zur Verwendung nach Anspruch 1, wobei das inhalierbare Stickstoffmonoxid in einer Dosis im Bereich von 25 Mikrogramm ($\mu$g)/kg Idealkörpergewicht (IBW)/Stunde bis 50 $\mu$g/kg IBW/Stunde verabreicht wird.

3. iNO zur Verwendung nach Anspruch 2, wobei das inhalierbare Stickstoffmonoxid in einer Dosis im Bereich von 30 $\mu$g/kg IBW/h bis 45 $\mu$g/kg IBW/h verabreicht wird.

4. iNO zur Verwendung nach Anspruch 2, wobei das inhalierte Stickstoffmonoxid in einer Dosis von 30 $\mu$g/kg IBW/h verabreicht wird.

5. iNO zur Verwendung nach Anspruch 2, wobei das inhalierte Stickstoffmonoxid in einer Dosis von 45 $\mu$g/kg IBW/h verabreicht wird.

6. iNO zur Verwendung nach einem der Ansprüche 1 bis 5, wobei die Aktivitätsniveaus im Vergleich zu einem Basisaktivitätsniveau verbessert oder aufrechterhalten werden, wobei die Aktivitätsniveaus einen oder mehrere Aktivitätsparameter umfassen, die unter Gesamtaktivität, nicht sitzender Aktivität, moderater Aktivität, moderater bis intensiver körperlicher Aktivität (MVPA), Schritten, Kalorien, metabolischen Äquivalenteinheiten (MET), Schlaf, Herzfrequenz, Sauerstoffsättigung und verbrannten Kalorien ausgewählt sind.

7. iNO zur Verwendung nach Anspruch 6, das ferner das Messen von Änderungen der Aktivitätsniveaus unter Verwendung von Aktigraphie umfasst.

8. iNO zur Verwendung nach einem der Ansprüche 1 bis 7, wobei der Algorithmus einen Schwellenwertsensitivitätsund/oder einen Steigungsalgorithmus verwendet, wobei der Steigungsalgorithmus einen Atemzug erkennt, wenn die Druckabfallrate einen vorgegebenen Schwellenwert erreicht.

9. iNO zur Verwendung nach einem der Ansprüche 1 bis 8, wobei die interstitielle Lungenerkrankung (ILD) einen oder mehrere Subtypen umfasst, die aus idiopathischer interstitieller Pneumonie (IIP), chronischer Hypersensitivitätspneumonie, berufs- oder umweltbedingter Lungenerkrankung, idiopathischer Lungenfibrose (IPF), Nicht-IPF-IIPs, granulomatöser und bindegewebserkrankungsbedingter ILD ausgewählt sind.

**10.** iNO zur Verwendung nach einem der Ansprüche 1 bis 8, wobei die interstitielle Lungenerkrankung eine idiopathische Lungenfibrose (IPF) umfasst.

**11.** iNO zur Verwendung nach einem der Ansprüche 1 bis 10, wobei der Patient ein hohes Risiko für die Entwicklung einer pulmonalen Hypertonie aufweist.

**Revendications**

**1.** Oxyde nitrique inhalé (iNO) pour son utilisation dans un procédé pour améliorer ou maintenir des niveaux d'activité chez un patient en ayant besoin, dans lequel le procédé comporte l'administration d'iNO au patient, dans lequel le patient présente une maladie pulmonaire interstitielle (ILD), dans lequel l'iNO est administré au patient par :

(a) détection d'un schéma respiratoire chez le patient incluant un temps inspiratoire total ;
(b) corrélation du schéma respiratoire avec un algorithme pour calculer un moment d'administration d'une dose d'oxyde nitrique ; et
(c) administration de la dose d'oxyde nitrique au patient de manière pulsatile sur une partie du temps inspiratoire total.

**2.** iNO selon la revendication 1, dans lequel l'oxyde nitrique inhalé est administré à une dose dans une plage de 25 microgrammes (mcg)/kg de poids corporel idéal (IBW)/h à 50 mcg/kg d'IBW/h.

**3.** iNO pour son utilisation selon la revendication 2, dans lequel l'oxyde nitrique inhalé est administré à une dose dans une plage de 30 mcg/kg d'IBW/h à 45 mcg/kg d'IBW/h.

**4.** iNO pour son utilisation selon la revendication 2, dans lequel l'oxyde nitrique inhalé est administré à une dose de 30 mcg/kg d'IBW/h.

**5.** iNO pour son utilisation selon la revendication 2, dans lequel l'oxyde nitrique inhalé est administré à une dose de 45 mcg/kg d'IBW/h.

**6.** iNO pour son utilisation selon l'une quelconque des revendications 1-5, dans lequel les niveaux d'activité sont améliorés ou maintenus par rapport à un niveau d'activité de référence, dans lequel les niveaux d'activité comportent un ou plusieurs paramètres d'activité sélectionnés parmi les activité globale, activité non sédentaire, activité modérée, activité physique modérée à vigoureuse (MVPA), pas, calories, unités métaboliques équivalentes (MET), sommeil, fréquence cardiaque, saturation en oxygène, et calories brûlées.

**7.** iNO pour son utilisation selon la revendication 6, comportant en outre la mesure des changements de niveaux d'activité à l'aide de l'actigraphie.

**8.** iNO pour son utilisation selon l'une quelconque des revendications 1-7, dans lequel l'algorithme utilise l'un ou les deux d'une sensibilité de seuil et d'un algorithme de pente, dans lequel l'algorithme de pente détecte une respiration lorsque le taux de chute de pression atteint un seuil prédéterminé.

**9.** iNO pour son utilisation selon l'une quelconque des revendications 1-8, dans lequel la maladie pulmonaire interstitielle (ILD) comporte un ou plusieurs sous-types choisis parmi les pneumonie interstitielle idiopathique (IIP), pneumonie d'hypersensibilité chronique, maladie pulmonaire professionnelle ou environnementale, fibrose pulmonaire idiopathique (FPI), IIP non-PFI, ILD granulomateuse et liée à une maladie du tissu conjonctif.

**10.** iNO pour son utilisation selon l'une quelconque des revendications 1-8, dans lequel la maladie pulmonaire interstitielle comporte une fibrose pulmonaire idiopathique (FPI) .

**11.** iNO pour son utilisation selon l'une quelconque des revendications 1-10, dans lequel le patient présente un risque élevé de développer une hypertension pulmonaire.

27

FIG. 1

EP 3 906 077 B1

FIG. 2

FIG. 3

Breath Detection Comparison

FIG. 4

FIG. 5A

FIG. 5B

FIG. 6A

FIG. 6B

FIG. 6C

FIG. 7A

FIG. 7B

FIG. 7C

FIG. 7D

EP 3 906 077 B1

FIG. 8A

FIG. 8B

# Normalized MVPA - Change from baseline

FIG. 9A

**Overall Activity - Change from baseline**

··□·· iNO45    ■ Placebo

* Data points and error bars = mean and standard error
* Cohort 2: n=44; randomized 2:1

FIG. 9B

EP 3 906 077 B1

SGRQ Total

SGRQ Total

- Data points and error bars = mean and standard error
- Change from baseline at month 4
- Cohort 2: n=44; randomized 2:1

FIG. 10A

FIG. 10B

## SGRQ Impacts

FIG. 10C

- Data points and error bars = mean and standard error
- Change from baseline at month 4
- Cohort 2: n=44; randomized 2:1

UCSD SOBQ

FIG. 11

FIG. 12B

FIG. 12A

FIG. 13A

FIG. 13B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019222640 A
- US 7523752 B
- US 8757148 B
- US 8770199 B
- US 8803717 B
- US 20130239963 A
- US 20160106949 A

**Non-patent literature cited in the description**

- **BLANCO et al.** *J. Appl. Physiol.*, 23 December 2010, vol. 110 (3), 638-645